(19) Europäisches Patentamt
European Patent Office
Office européen des brevets

(11) **EP 2 407 097 B1**

(12) **EUROPEAN PATENT SPECIFICATION**

(45) Date of publication and mention
of the grant of the patent:
**06.05.2020  Bulletin 2020/19**

(51) Int Cl.:
***A61B 5/0452*** (2006.01)

(21) Application number: **11172371.4**

(22) Date of filing: **01.07.2011**

(54) **Method and apparatus for noise detection in physiological signals**

Verfahren und Vorrichtung zur Rauscherfassung in physiologischen Signalen

Procédé et appareil de détection sonore dans des signaux physiologiques

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.07.2010  US 363669 P**
**28.03.2011  US 468074 P**

(43) Date of publication of application:
**18.01.2012  Bulletin 2012/03**

(73) Proprietor: **BIOTRONIK SE & Co. KG**
**12359 Berlin (DE)**

(72) Inventors:
• **Lian, Jie**
**Beaverton, OR Oregon 97007 (US)**
• **Müssig, Dirk**
**West Linn, OR Oregon 97068 (US)**

(74) Representative: **Galander, Marcus et al**
**Biotronik Corporate Services SE**
**Corporate Intellectual Property**
**Sieversufer 7 - 9**
**12359 Berlin (DE)**

(56) References cited:
**EP-A2- 2 220 995      US-A- 5 891 048**
**US-A1- 2004 049 120      US-A1- 2007 255 150**
**US-A1- 2008 269 813      US-A1- 2008 281 216**
**US-B1- 6 230 059      US-B1- 6 321 115**
**US-B1- 6 345 199**

**Description**

The present invention generally relates to medical devices that measure and analyze the cardiac electrical signals. More particularly, the present invention relates to a method and apparatus for quantifying signal quality from electrocardiogram (ECG) surface electrocardiogram, subcutaneous electrocardiogram, or intracardiac electrogram signals. The invention further relates to medical devices for processing sampled physiological signals and in particular to devices having means for sensing electrocardiogram (ECG) surface electrocardiogram, subcutaneous electrocardiogram, or intracardiac electrogram signals and a processing unit, the processing unit being adapted to determine the quality of the signal. Irrespective of ECG recording apparatus (e.g., ECG machines, bedside ECG monitors, Holter ECG monitors, subcutaneous ECG devices, implantable cardiac pacemakers, etc.), reliable beat detection is the prerequisite for further ECG processing and clinical diagnosis. Beat detection may involve the detection of signal peaks that exceed a preset threshold and that presumably represent heart contractions. However, the accuracy of ECG beat detection is often compromised by noise from many different sources, such as high frequency muscle noise and electromagnetic interference (EMI), low frequency noise due to respiration, saturation noise due to charge overloading of sense amplifiers, and so on. Advanced filter design can only reduce but not eliminate these noises.

Noise in an ECG signal can cause two types of beat detection errors. False positive (FP) occurs when beat detection algorithm falsely generates a sense marker when there is no QRS complex, and false negative (FN) occurs when beat detection algorithm fails to detect the true QRS complex. Although it is well known that large noise can cause FPs and small signal can lead to FNs, it is important to realize that FPs can also occur at low noise level and FNs can also occur when signal amplitude is high. For example, many ECG beat detection algorithms automatically apply a blanking window after each beat detection and then dynamically adjust the sensing threshold (one exemplary Auto-Sensing algorithm has been disclosed in U.S. Pat. No. 5,891,048, assigned to the present assignee). When the sensing threshold becomes too low, a sample with small noise amplitude may trigger a false detection (i.e. FP). On the other hand, each false detection triggers a blanking window during which a true QRS may be missed from detection (i.e. FN). Therefore, for reliable ECG beat detection, a high signal-to-noise ratio (SNR) is desired. In other words, when SNR is low, ECG beat detections may be unreliable.

Both FPs and FNs can cause problems for ECG rhythm analysis. For example, FPs and FNs can lead to irregular RR intervals, which could be misinterpreted by the device as atrial fibrillation. In another example, frequent FPs may lead to very short RR intervals, which could be misdiagnosed by the device as ventricular tachyarrhythmia. Yet in another example, consecutive FNs may generate very long RR intervals, which could be misdiagnosed by the device as bradycardia or ventricular asystole. As a result, physicians may have to spend tremendous amount of time and effort to examine these false rhythm classifications. Moreover, for ECG loop recorders, these false rhythm alarms could overwhelm the device's memory, which otherwise could have been used to store more useful arrhythmic episodes.

In US 7,496,409 issued to Greenhut et al, the signal quality of the subcutaneous ECG is measured by a plural of metrics including R wave amplitude, a signal-to-noise ratio such as an R-wave peak amplitude to a maximum or average waveform amplitude between R-waves or an R-wave to T-wave amplitude ratio, a signal slope or slew rate, a low slope content, a relative high versus low frequency power, mean frequency or spectral width estimation, probability density function, normalized mean rectified amplitude, or any combination of these metrics. These metrics either require high computation power and is not suitable for implementation in implantable devices, or have unsatisfactory performance to characterize the noise conditions.

In US 6,230,059 issued to Duffin, each stored episode data is checked for noise condition. Specifically, the most frequently occurring amplitude value (MCV) in the data set is determined, and a baseline amplitude value or range is derived from the MCV. The crossings of the baseline value are determined, and a crossing count of the total number of crossings of the baseline value is made. The crossing count is compared to a noise threshold count, and the EGM episode data is tagged as noisy if the crossing count exceeds or equals the noise threshold count or is tagged as noise free if the noise threshold count is less than the crossing count. However, there are several major limitations of this method. First, it can only be applied to a snapshot of episode data, and cannot be used for noise detection on the beat-by-beat basis. Second, the MCV is affected by the episode. When the episode is not representative of the quiescent ECG segments, the MCV may deviate from the true baseline. Third, the baseline range is arbitrarily defined without consideration of the signal amplitude. As a result, an ECG episode may be tagged as noisy despite its large signal-to-noise ratio (SNR), whereas another ECG episode may be tagged as noise-free despite its small SNR.

In US 7,467,009 and US 6,917,830 both issued to Palreddy et al, the presence or absence of noise is determined by computing the density of local peaks or inflection points in the ECG waveform. A sample is defined as the inflection point if its amplitude is greater, or smaller, than both its preceding and the following samples by predefined threshold values. Because the predefined threshold values are not adaptive to the signal amplitude, more inflection points may be detected for an ECG signal with high SNR than another ECG signal with small SNR.

US 7,474,247 issued to Hinks et al. disclosed a method to detect saturation noise by means of an integrator or quantizer in the front-end circuit, but does not address the more challenging task of detecting high-frequency noise introduced by muscle activities or EMI.

In US 7,027,858 issued to Cao et al., the noise markers are generated if the detection intervals are considered as too short to be physiologically valid. Similar concept was also disclosed in US 2008/0161873 by Gunderson, and in US 2008/0300497 by Krause et al. These interval-based noise detection methods are neither sensitive nor specific for noise detection.

US 2008/269813 by Greenhut e.al and US 2007/255150 by Brodnick disclose amplitude threshold based signal analysis for signal quality determination and noise detection. US 6345199 issued to Thong discloses amplitude threshold based signal analysis in combination with morphology analysis for signal discrimination.

US 7,570,989 issued to Baura et al. disclosed a method which uses the change in RR interval from the previous beat, and the change in mean squared error (MSE) from the last n (e.g. n=2) good beats, to determine whether the beat under analysis should be further processed. The MSE change and the RR interval change could be caused by many factors other than noise, so this method is not reliable for noise detection.

US 5,999,845 issued to dePinto disclosed a method to detect wideband noise by measuring the running average of the squared sample values of the ECG signal with QRS complex blanked.

US 5,647,379 issued to Meltzer disclosed a method to detect the level of EMI component of an input biomedical signal by calculating a correlation function. However, none of these methods has proven accuracy in quantifying the ECG signal quality.

In US 2010/0312131 A1, Naware et al. disclosed a method for noise detection by counting the number of noise threshold crossings in a noise detection window, where the noise threshold is preferably a specified percentage of the beat detection sensing thresh-old. This method has several shortcomings. First, the beat detection sensing threshold may or may not be adaptive to the signal amplitude. Hence there is no guarantee that the noise threshold reflects the signal amplitude, thus the method does not take into account the signal to noise ratio in noise detection. Second, the noise window is either fixed or back-to-back, thus the method does not allow sample-by-sample or real-time noise detection. Third, the noise detection window is triggered by sensed ventricular events. Therefore, under-sensing of ventricular events will not trigger noise detection, thus leading to under-detection of noises. On the other hand, over-sensing of large artefact may lead to too high noise threshold, which can also lead to under-detection of noises. US 6,321,115 issued to Mouchawar et al. disclosed a method for detecting noise in an electrogram based on crossing detection of a positive and negative threshold. Other relevant prior art includes US 2008/0281216 A1.

In view of above, there is a need to provide a method for more reliable detection of noise in physiological signals, so that noisy beats and intervals are excluded from the rate and rhythm analysis by the device. There is also a need for an implantable device having a processing unit which implements a method for more reliable detection of noise.

According to a first aspect, as defined in claim 1, the invention is directed to a computer-implemented method for determination of a noise in a sampled cardiac electrical signal, said sampled cardiac electrical signal comprising a consecutive sequence of samples, each sample having an amplitude, the amplitudes in general being positive or negative, the method comprising the steps of

- providing a signal quality check window that comprises a number of consecutive samples

- providing at least one upper threshold for a signal amplitude

- providing at least one lower threshold for a signal amplitude, said at least one upper threshold and said at least one lower threshold defining at least three amplitude ranges, a high range for signal amplitude exceeding said upper threshold, a medium range for amplitudes between said upper threshold and said lower threshold and a low range for amplitude below said lower threshold

- comparing each sample within said quality check window with said upper threshold and with said lower threshold; and

- performing a signal quality analysis for said signal sample by analyzing the changes in amplitude of successive samples within said quality check window, said change in amplitude being determined for any two adjacent sample pairs within said quality check window by comparing to which amplitude ranges the amplitudes of the signal samples of the respective sample pair belong to.

The method further comprises determining a threshold crossing number (also referred to as number of noise threshold-crossings (NZX_CNT)) reflecting the number of thresholds between each two adjacent signal samples of each sample pair.

The method preferably further comprises determining a noise threshold crossing sum of said threshold crossing numbers for a respective quality check window. According to one preferred embodiment, the noise threshold crossing sum preferably is a weighted sum with predetermined weights for each threshold crossing number magnitude.

EP 2 407 097 B1

According to the method aspect of the invention, the noise condition is continuously evaluated on a sample-by-sample base e.g. by moving the noise detection window. The amplitudes of the samples can be either positive or negative, however the invention can also be applied for signals either having only positive or only negative values. In the latter case, providing only one noise threshold may be sufficient.

The number of samples a respective quality check window (QCW) (also referred to a noise detection window NZDW) contains, the at least one upper threshold (NZTHRESH or HITHRESH and LOTHRESH) and the at least one lower threshold (-NZTHRESH or - HITHRESH and -LOTHRESH) are in general parameters that can either be fixed or updated during operation. Each sample is associated with the quality check window (or noise detection window) that has the length of the sample number (QCW or NZDW, respectively).

The cardiac electrical signal is either an electrocardiogram (ECG) signal, a surface electrocardiogram signal, a subcutaneous electrocardiogram signal or a intracardiac electrogram signal. The electrocardiogram (ECG) signal can also be derived from an impedance signal, an transthoracic impedance signal, an intracardiac impedance signal or any other electrical signal reflecting cardiac activity.

In a preferred embodiment, the cardiac electrical signal is an electrocardiogram (ECG) signal.

According to one embodiment, the magnitudes of the at least one upper threshold and the at least one lower noise threshold are identical. According to a further embodiment, the at least one upper threshold is positive and the at least one lower threshold is negative. These two embodiments, which can be implemented in parallel, are especially preferable if the physiological signal has a mean value of almost zero, or, in other words, a zero baseline.

In order to detect beats and/or QRS complexes, an ECG beat detection can be performed by determining if an amplitude of any sample exceeds a beat detection threshold, and generating a sense marker at this sample. The value of the beat detection threshold can be predefined and is either fixed or continuously updated during operation. Preferably, a peak detection window (PKDW) around the sense marker, having predefined length and position with respect to the sense marker, is defined, and a maximum absolute amplitude of all samples in the peak detection window (PKDW) is determined as a peak value (PEAK). This yields an indication for the peak value of a QRS complex.

In a preferred embodiment, the at least one upper threshold and/or the at least one lower threshold are recalculated after each ECG beat detection. This allows for an adaptation of the thresholds to ambient conditions that could change with time.

The noise threshold can be kept constant until the next beat detection, or can be lowered to a predefined minimum noise threshold when no beat is detected after a predefined duration.

Preferably, the at least one upper noise threshold is recalculated after an ECG beat detection, each ECG beat detection yielding a peak value (PEAK) corresponding to a maximum absolute amplitude measured during a peak detection window (PKDW), by performing the following steps:

(a) calculating a minimum value (NEW PEAK) by taking the minimum of a peak value (PEAK) of a recently performed ECG beat detection, and a weighted average value (OLD PEAK) calculated directly after the previous ECG beat detection and being scaled by a first predefined scaling factor (p) larger than one,

(b) calculating a weighted average value (OLD_PEAK) of the minimum value (NEW PEAK) calculated in step (a), and the weighted average value (OLD PEAK) calculated directly after the previous ECG beat detection, the minimum value (NEW PEAK) and the weighted average value (OLD PEAK) each being weighted by respective predefined weighting factors (w1, w2), the predefined weighting factors (w1, w2) summing to one, and

(c) recalculating the at least one upper threshold by taking the maximum of either a predefined minimum threshold value, or the weighted average value (OLD PEAK) calculated in step (b) and being scaled by a second predefined scaling factor (f), the second predefined scaling factor (f) being larger than zero and smaller than one.

With these steps it is ensured that the upper noise threshold does not fall below the minimum threshold value in order to omit that too many samples are erroneously qualified as noisy. It further provides for a smooth adaptation to changing peak values (PEAK), i.e. a sudden change in the peak value (PEAK) does not lead to an immediate change of the upper noise threshold by the same order of magnitude. This is advantageous if a single peak has an unnaturally high value due to certain adverse effects.

The at least one lower threshold can be adapted accordingly, especially it can be set to the negative of the the at least one upper threshold.

According to a first preferred embodiment, the method comprises the steps of:

- providing a sample number (NZDW), an upper noise threshold (NZTHRESH) and a lower noise threshold (-NZTHRESH),
- defining a noise detection window comprising a number (NZDW) of samples including a last sample (k), said number corresponding to said sample number,

- determining a supra-threshold sample sum (STS_SUM) corresponding to the number of samples in said noise detection window that have an amplitude that either exceeds said upper noise threshold (NZTHRESH) or falls below said lower noise threshold (-NZTHRESH), respectively,
- determining a noise threshold crossing sum (NZX SUM) corresponding to the sum of the number of noise threshold-crossings (NZX CNT) between each two consecutive samples in the noise detection window,
- indicating a noise condition for each last sample (k) of each noise detection window, if

    - said noise threshold crossing sum (NZX_SUM) exceeds a first predefined value (TX1), or
    - said supra-threshold sample sum (STS_SUM) exceeds a second predefined value (ST1), or
    - said noise threshold crossing sum (NZX_SUM) exceeds a third predefined value (TX2) and said supra-threshold sample sum (STS_SUM) exceeds a fourth predefined value (ST2).

The noise threshold crossing sum (NZX SUM) is a measure for the occurrences of crossings of the upper noise threshold (NZTHRESH) and the lower noise threshold (-NZTHRESH) in between each two consecutive samples. If the amplitudes of two consecutive samples in the noise detection window either both exceed the upper noise threshold (NZTHRESH), both lie between the upper noise threshold (NZTHRESH) and the lower noise threshold (-NZTHRESH), or both fall below the lower noise threshold (-NZTHRESH), the noise threshold crossing sum (NZX_SUM) remains unchanged. If the amplitude of one of two consecutive samples exceeds the upper noise threshold (NZTHRESH) and the amplitude of the other one lies between the upper noise threshold (NZTHRESH) and the lower noise threshold (-NZTHRESH), the noise threshold crossing sum (NZX SUM) is increased by one. If the amplitude of one of two consecutive samples falls below the lower noise threshold (-NZTHRESH) and the amplitude of the other one lies between the upper noise threshold (NZTHRESH) and the lower noise threshold (-NZTHRESH), the noise threshold crossing sum (NZX SUM) is also increased by one. If the amplitude of one of two consecutive samples exceeds the upper noise threshold (NZTHRESH) and the amplitude of the respective other sample falls below the lower noise threshold (-NZTHRESH), the noise threshold crossing sum (NZX_SUM) is increased by two. Thus, the noise threshold crossing sum (NZX SUM) indicates how often the first threshold (NZTHRESH) or the second threshold (-NZTHRESH), respectively, fall between amplitudes of consecutive samples in the noise detection window.

The noise condition is indicated for the last sample (k) of each noise detection window, if either the noise threshold crossing sum (NZX_SUM) exceeds a first predefined value (TX1), or the supra-threshold sample sum (STS_SUM) exceeds a second predefined value (ST1), or the noise threshold crossing sum (NZX_SUM) exceeds a third predefined value (TX2) and the supra-threshold sample sum (STS_SUM) exceeds a fourth predefined value (ST2). Thus, the method according to the first aspect of the invention provides for a simple and reliable detection of the noise condition for each sample (k) out of a sequence of samples.

For calculating the supra-threshold sample sum (STS_SUM), each sample in the noise detection window (NZDW) is evaluated in terms of its amplitude. If the amplitude lies between the upper noise threshold (NZTHRESH) and the lower noise threshold (-NZTHRESH), the supra-threshold sample sum (STS_SUM) remains unchanged. If the amplitude either exceeds the upper noise threshold (NZTHRESH) or falls below the lower noise threshold (-NZTHRESH), the supra-threshold sample sum (STS_SUM) is increased by one.

Determining the noise condition for the last sample of the noise detection window allows for determining the noise condition in real time for each new sample on a moving window basis. This is, because each noise detection window comprises a new sample and the NZDW-1 samples immediately preceding the new sample. As a consequence, the inventive method allows for real time noise detection for each new sample.

In a preferred embodiment, when a low signal quality is indicated for any given sample, the detected beats immediately before and immediately after this sample are labeled as unreliable. The interval between this pair of unreliable detections can be labeled as noisy interval. In another embodiment, signal quality is evaluated on a moving window basis. The moving window consists of a predefined number Y of samples. When for another predefined number X out of Y samples a low signal quality is indicated, the noise condition is confirmed. Then the detected beats immediately before and immediately after this window are labeled as unreliable. The interval between this pair of unreliable detections can be labeled as noisy interval.

According to a further preferred embodiment of the method, the weighted noise threshold crossing sum is a complexity index (CI), said method further comprising the steps of comparing said complexity index with a predefined complexity index threshold and generating a low signal quality indicating signal if said complexity index exceeds said complexity index threshold.

According to a second aspect, the invention is directed to a medical device for processing a sampled cardiac electrical signal according to claim 13. Preferably, the medical device is an implantable device having means for sensing an electrocardiogram (ECG) signal and a processing unit, said electrocardiogram (ECG) signal comprising a consecutive sequence of samples, each sample having an amplitude, the amplitudes in general being positive or negative, wherein the processing unit is configured to determine a noise condition of a sample (k) by performing the steps of

- providing a sample number (NZDW), an upper noise threshold (NZTHRESH) and a lower noise threshold (-NZTHRESH),
- defining a noise detection window comprising a number (NZDW) of samples including a last sample (k), said number corresponding to said sample number,
- determining a supra-threshold sample sum (STS_SUM) corresponding to the number of samples in said noise detection window that have an amplitude that either exceeds said upper noise threshold (NZTHRESH) or falls below said lower noise threshold (-NZTHRESH), respectively,
- determining a noise threshold crossing sum (NZX SUM) corresponding to the sum of the number of noise threshold-crossings (NZX CNT) between each two consecutive samples in the noise detection window,
- indicating a noise condition for each last sample (k) of each noise detection window, if

    - said noise threshold crossing sum (NZX_SUM) exceeds a first predefined value (TX1), or
    - said supra-threshold sample sum (STS_SUM) exceeds a second predefined value (ST1), or
    - said noise threshold crossing sum (NZX_SUM) exceeds a third predefined value (TX2) and said supra-threshold sample sum (STS_SUM) exceeds a fourth predefined value (ST2).

The implantable device according to the second aspect basically performs the method according to the first aspect with an ECG signal which it senses itself. The noise condition is continuously evaluated on a sample-by-sample base. The amplitudes of the samples can be both positive or negative, however the invention can also be applied for signals either having only positive or only negative values. In the latter case, providing only one noise threshold may be sufficient.

The sample number (NZDW), the upper noise threshold (NZTHRESH) and the lower noise threshold (-NZTHRESH) are in general parameters that can either be fixed or updated during operation. Each sample is associated with a noise detection window that has the length of the sample number (NZDW).

For calculating the supra-threshold sample sum (STS_SUM), each sample in the noise detection window (NZDW) is evaluated in terms of its amplitude. If it lies between the upper noise threshold (NZTHRESH) and the lower noise threshold (-NZTHRESH), the supra-threshold sample sum (STS_SUM) remains unchanged. If it either exceeds the upper noise threshold (NZTHRESH) or falls below the lower noise threshold (-NZTHRESH), the supra-threshold sample sum (STS_SUM) is increased by one.

The noise threshold crossing sum (NZX SUM) is a measure for the occurrences of crossings of the upper noise threshold (NZTHRESH) and the lower noise threshold (-NZTHRESH) in between each two consecutive samples. If the amplitudes of two consecutive samples in the noise detection window either both exceed the upper noise threshold (NZTHRESH), both lie between the upper noise threshold (NZTHRESH) and the lower noise threshold (-NZTHRESH), or both fall below the lower noise threshold (-NZTHRESH), the noise threshold crossing sum (NZX_SUM) remains unchanged. If the amplitude of one of two consecutive samples exceeds the upper noise threshold (NZTHRESH) and the amplitude of the other one lies between the upper noise threshold (NZTHRESH) and the lower noise threshold (-NZTHRESH), the noise threshold crossing sum (NZX_SUM) is increased by one. If the amplitude of one of two consecutive samples falls below the lower noise threshold (-NZTHRESH) and the amplitude of the other one lies between the upper noise threshold (NZTHRESH) and the lower noise threshold (-NZTHRESH), the noise threshold crossing sum (NZX SUM) is also increased by one. If the amplitude of one of two consecutive samples exceeds the upper noise threshold (NZTHRESH) and the amplitude of the other one falls below the lower noise threshold (-NZTHRESH), the noise threshold crossing sum (NZX SUM) is increased by two. Thus, the noise threshold crossing sum (NZX SUM) indicates how often the first threshold (NZTHRESH) or the second threshold (-NZTHRESH), respectively, fall between amplitudes of consecutive samples in the noise detection window.

The noise condition is indicated for the last sample (k) of each noise detection window, if either the noise threshold crossing sum (NZX_SUM) exceeds a first predefined value (TX1), or the supra-threshold sample sum (STS_SUM) exceeds a second predefined value (ST1), or the noise threshold crossing sum (NZX_SUM) exceeds a third predefined value (TX2) and the supra-threshold sample sum (STS_SUM) exceeds a fourth predefined value (ST2). Thus, the implantable device according to the second aspect of the invention can easily and reliably detect the noise condition for each sample (k) out of a sequence of samples.

According to one embodiment, the magnitudes of the upper noise threshold (NZTHRESH) and the lower noise threshold (-NZTHRESH) are identical. According to a further embodiment, the upper noise threshold (NZTHRESH) is positive and the lower noise threshold (-NZTHRESH) is negative. These two embodiments, which can be implemented in parallel, are especially preferable if the ECG signal has a mean value of almost zero.

Preferably, the processing unit is further configured to perform an ECG beat detection and/or a detection of a QRS complex. This can be done by determining if an amplitude of any sample exceeds a beat detection threshold, and generating a sense marker at this sample. The value of the beat detection threshold can be predefined and is either fixed or continuously updated during operation of the implantable device. Further preferable, the processing unit is further configured to determine a peak value (PEAK) associated with each ECG peak detection, by defining a peak detection window (PKDW) around the sense marker, having predefined length and position with respect to the sense marker, and determining a maximum absolute amplitude of all samples in the peak detection window (PKDW) as a peak value (PEAK). This yields an indication for the peak value of a QRS complex.

In a preferred embodiment, the processing unit is adapted to recalculate the upper noise threshold (NZTHRESH) and/or the lower noise threshold (-NZTHRESH) after each ECG beat detection. This allows for an adaptation of the thresholds to ambient conditions that could change with time.

The noise threshold can be kept constant until the next beat detection, or can be lowered to a predefined minimum noise threshold when no beat is detected after a predefined duration.

Preferably, the processing unit is configured to recalculate the upper noise threshold (NZTHRESH) after an ECG beat detection, each ECG beat detection yielding a peak value (PEAK) corresponding to a maximum absolute amplitude measured during a peak detection window (PKDW), by performing the following steps:

(a) calculating a minimum value (NEW_PEAK) by taking the minimum of a peak value (PEAK) of a recently performed ECG beat detection, and a weighted average value (OLD_PEAK) calculated directly after the previous ECG beat detection and being scaled by a first predefined scaling factor (p) larger than one,

(b) calculating a weighted average value (OLD PEAK) of the minimum value (NEW PEAK) calculated in step (a), and the weighted average value (OLD PEAK) calculated directly after the previous ECG beat detection, the minimum value (NEW PEAK) and the weighted average value (OLD PEAK) each being weighted by respective predefined weighting factors (w1, w2), the predefined weighting factors (w1, w2) summing to one, and

(c) recalculating the upper noise threshold (NZTHRESH) by taking the maximum of either a predefined minimum threshold value (MIN_NZTHRESH), or the weighted average value (OLD_PEAK) calculated in step (b) and being scaled by a second predefined scaling factor (f), the second predefined scaling factor (f) being larger than zero and smaller than one.

With these steps it is ensured that the upper noise threshold does not fall below the minimum threshold value (MIN NZTHRESH) in order to omit that too many samples are erroneously qualified as noisy. It further provides for a smooth adaptation to changing peak values (PEAK), i.e. a sudden change in the peak value (PEAK) does not lead to an immediate change of the upper noise threshold by the same order of magnitude. This is advantageous if a single peak has an unnaturally high value due to certain artefacts.

The lower noise threshold (-NZTHRESH) can be adapted accordingly, especially it can be set to the negative of the upper noise threshold (NZTHRESH).

In a preferred embodiment, when noise condition is indicated for any given sample, the detected beats immediately before and immediately after this sample are labeled as unreliable. The intervals associated with this pair of unreliable detections can be labeled as noisy intervals. In another embodiment, noise detection is evaluated on a moving window basis. The moving window consists of a predefined number Y of samples. When for another predefined number X out of Y samples the noise condition is indicated, the noise condition is confirmed. Then the detected beats immediately before and immediately after this window are labeled as unreliable. The intervals associated with this pair of unreliable detections can be labeled as noisy intervals.

The details of the invention can be understood from the following drawings and the corresponding text descriptions.

FIG. 1    shows a block diagram of an implantable subcutaneous ECG monitoring device, and its interfaces with an external programming device and an external portable device, which further communicates with a remote service center.

FIG. 2    illustrates the concept of sample-by-sample noise detection window (NZDW). The noise threshold (NZTHRESH) and the noise tolerance zone (NTZ) are also illustrated.

FIG. 3    illustrates the peak detection window (PKDW) around each beat detection, the adaptive noise threshold (NZTHRESH) and the associated noise tolerance zone (NTZ).

FIG. 4    illustrates the case that the NZTHRESH is changed to MIN_NZTHRESH when no beat is detected after the duration of NZTHRESH_HOLD.

FIG. 5A    illustrates the method of counting noise threshold crossings, and

FIG. 5B    illustrates the method of counting supra-threshold samples.

FIG. 6    shows an example according to a preferred embodiment to flag noise samples, unreliable detections, and the noisy interval.

FIG. 7    shows an example according to another embodiment to flag noise samples, unreliable detections, and the noisy intervals.

FIG. 8    shows a typical example of ECG noise detection result.

FIG. 9    illustrates the concept of sample-by-sample quality check window (QCW) and the space segmentation by the high threshold (HITHRESH) and low threshold (LOTHRESH) vectors.

FIG. 10    illustrates the peak detection window (PKDW) around each beat detection, the adaptive HITHRESH and LOTHRESH vectors and the associated subspaces.

FIG. 11    illustrates the case that the HITHRESH and LOTHRESH are changed to their respective minimum values when no beat is detected after the duration of THRESH_HOLD.

FIG. 12    illustrates the method of converting signal samples to symbols, and translating the signal dynamics to symbol change steps.

FIG. 13    illustrates the method of constructing a histogram of symbol change step for a given QCW.

FIG. 14    shows a typical example of ECG recording with burst of noise and the calculated complexity index which can be used to quantify the signal quality.

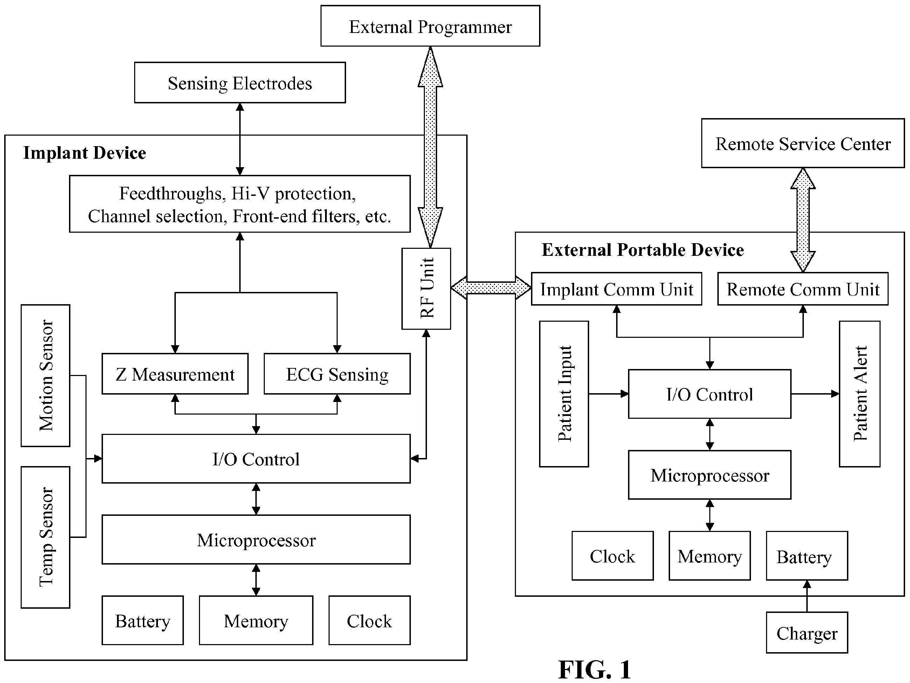

Figure 1 shows a block diagram of an implantable device for subcutaneous ECG monitoring, and its interfaces with an external programmer and an external portable device, which further communicates with a remote service center.
Refer to Figure 1. The implantable device comprises an electronic circuitry that is hermetically sealed inside a Can, which is made from a biocompatible conductive material such as titanium, an optional non-conductive header attached to the Can, three or more sensing electrodes, with or without leads connected to the header.
The sensing electrodes, which are electrically isolated from one another, are mounted over the outer surface of the Can, or outside the header (if available), or at the distal end of the leads (if available). For subcutaneous ECG recording, one or more pairs of sensing electrodes form the sensing vectors and the inter-electrode distance is preferably greater than 3 cm.
The leads are optional for subcutaneous ECG recording. Generally, if the measured subcutaneous ECG amplitude is too small for reliable sensing, despite configuring different sensing vectors and recording at different anatomical locations, then one or more subcutaneous leads (with distal electrodes) could be tunneled under the patient's skin and connected to the header, so that a larger subcutaneous ECG amplitude could be measured by increasing inter-electrode distance, e.g., between the lead electrode and the Can or header electrode.
Still refer to Figure 1. Enclosed inside the hermetically sealed Can, a microprocessor and associated circuitry make up the controller of the implant device. The implant device is powered by a battery, and maintains an internal clock for timing the operations. The microprocessor communicates with a memory via a bi-directional data bus. The memory typically comprises a ROM or RAM for program storage and a RAM for data storage.
The sensing electrodes are first connected to an electronic interface that preferably includes a feedthrough circuitry for noise reduction, a high voltage protection circuitry, a switch network circuitry for sensing channel selection, and front-end analog filters, as well known in the field. The configurations of the interface circuitry (e.g., filter settings, sensing channel selection, etc.) can be programmed by the microprocessor.
The microprocessor connects to an I/O control unit to manage the input and output of the implant device. One input signal is the subcutaneous ECG picked up by the sensing electrodes. After pre-processed by the interface circuitry, the subcutaneous ECG signal is further processed by the ECG sensing unit, which usually comprises amplifiers, analog-to-digital converters, digital filters, etc., as known in the art. By way of the analog to digital converting the physiological signal is sampled.
Another input signal is the impedance (Z) signal measured between the sensing electrodes. By injecting a small constant current (e.g., $100\mu A$, preferably biphasic) between two electrodes while measuring the voltage difference between the same or different pair of electrodes, the impedance is calculated as the ratio between the measured voltage difference and the injecting current strength. As known in the art, the impedance signal provides useful information on the integrity of the sensing channel. In addition, the continuously measured impedance signal may be further processed by the microprocessor to extract other physiological status of the patient, such as the respiration rate.

Other types of physiological signals measured by specific sensors can also serve as input to the implant device. For example, an on-board accelerometer can serve as a motion sensor that provides patient's activity signal to the implant device, and an on-board (or embedded in the lead) temperature sensor can provide the subcutaneous temperature signal to the implant device. Other types of input signals include, but are not limited to, the subcutaneous pressure signal measured by a pressure sensor, the acoustic signal measured by an acoustic sensor, the subcutaneous pH signal measured by a pH sensor, etc.

By running the program stored in the memory, the microprocessor also sends instructions to the ECG sensing unit, the impedance measurement unit, and other input measurement units to control how these signals are acquired (e.g., gain, offset, filter settings, sampling frequency, sampling resolution, etc.).

The acquired physiological signals are then stored in the device memory according to predefined storage modes. One typical mode is the queue-loop mode, meaning the acquired signals are stored in a predefined memory space, and while the allocated memory space is full, the newly acquired signals replace the oldest stored data. Another typical mode is the snapshot mode, meaning the acquired signals are stored in a predefined memory space, and while the allocated memory space is full, the newly acquired signals are not stored until the microprocessor decides to store another episode of data. Yet another typical mode is the mixed mode, in which one or more segments of allocated memory space store the acquired signals in queue-loop mode, whereas one or more segments of separately allocated memory space store the data in snapshot mode.

The acquired physiological signals are analyzed by the microprocessor by running programmed algorithms. For example, the microprocessor continuously analyzes the acquired subcutaneous ECG signals to detect the peak of QRS complexes. Such QRS peak detection can be achieved by many different means. One typical embodiment is to use an Auto-Sensing algorithm that applies a detection hold-off period after each peak detection, then automatically adjusts the sensing threshold, which is adaptive to the measured peak amplitude of the QRS complex and varies based on predetermined time dependence. One exemplary Auto-Sensing algorithm has been disclosed in US 5,891,048, assigned to the present assignee.

Accordingly, the implant device measures the intervals between any two adjacent peaks of the detected QRS complexes, and these intervals are termed RR intervals. These measured RR intervals are stored in the device memory. Similarly, the microprocessor can also continuously analyze the acquired subcutaneous ECG signals to measure other metrics of the QRS complex, such as the width of the QRS complex, the positive or negative peak amplitude of the QRS complex, the absolute area under the QRS complex, the maximum positive or negative slopes of the QRS complex, the dominant frequency component of the QRS complex, the complexity measures (e.g., sampled entropy) of the QRS complex, and so on. Likewise, the time series of these measured metrics are stored in the device memory for further analysis.

The implant device also includes a radio-frequency (RF) telemetry unit. The RF telemetry unit may be of the type well known in the art for conveying various information which it obtains from the implant device to the external programmer, or for receiving programming parameters from the external programmer and then conveying to the implant device. In one typical embodiment, the external programmer can interrogate the implant device to get the status of the implant device (e.g., battery status, sensing channel impedance, etc.) or the data recorded by the implant device (e.g., peak amplitude of the QRS complexes, statistics of measured RR intervals, etc.). In another typical embodiment, the external programmer can be used to activate or deactivate selected algorithms or update programmable parameters of the implant device.

In addition, the external portable device to be described hereinafter, can also communicate bi-directionally with the implant device through the telemetry unit. Preferably, the data that may be received from or sent to the external portable device are more limited as compared to the data that may be received from or sent to the external programmer.

In a preferred embodiment, the data that are transmitted from the external portable device to the implant device are simple commands, such as trigger a snapshot of the acquired subcutaneous ECG, retrieve most recently diagnostic information from the implanted device, etc. These commands set the implant device into one of a number of modalities wherein each modality is determined and controlled by parameters that can only be selected by a physician operating the external programmer using secure password or codes.

The data that are transmitted from the implant device to the external portable device preferably include simple acknowledgment to confirm receiving the commands from the external portable device, the signals warning the detection of abnormal conditions, such as detection of atrial fibrillation (AF), detection of high ventricular rate (HVR), detection of low ventricular rate (LVR), detection of abnormal sensing impedance, detection of abnormal temperature, and so on. Other diagnostic information, such as the AF burden, the frequency of ectopic beats, snapshots of RR intervals or subcutaneous ECG, etc., can also be transmitted to the external portable device. Preferably, a physician operating the external programmer using secure password or codes controls the enable or disable condition as well as the amount of data that can be transmitted from the implant device to the external portable device.

Still refer to Figure 1. The external portable device has a power source, such as a lithium battery, which provides power to the electrical components of the device. The battery is rechargeable by connecting to an external charger. The external portable device also maintains an internal clock for timing its operations. The overall functioning of the external portable device is controlled by its microprocessor, which reads and performs instructions stored in its associated memory. The instructions stored in memory preferably include instructions defining a communication protocol compatible with the implant device, and instructions defining a communication protocol compatible with the remote service center.

The microprocessor of the external portable device communicates with an I/O control unit to read from the keypad (or press switches) the patient input commands. In an exemplary embodiment, one subset of the input commands is designed to configure the external portable device, for example, to turn on or off certain outputs as described hereinafter, or select specific communication protocols. Another subset of the input commands is designed to establish communication between the external portable device and the remote service center through a remote communication unit. For example, patient's input can command the external portable device to transmit diagnostic information (retrieved from the implant device) to the remote service center, and wait to receive acknowledgment. The third subset of the commands is designed to establish communication between the external portable device and the implant device through the implant communication unit. For example, patient's input can command the external portable device to transmit corresponding signals to the implant device to trigger recording a snapshot of the subcutaneous ECG, to retrieve diagnostic information from the implant device, etc. The implant communication unit also receives the acknowledgment and related diagnostic information sent from the implant device, and conveys these data to the microprocessor for storage in the memory.

According to one exemplary embodiment of the present invention, upon receiving a predefined warning signal from the implant device (e.g., detection of AF, detection of HVR, detection of LVR, detection of abnormal sensing impedance, detection of abnormal temperature, etc.), the microprocessor of the external portable device communicates with the I/O control unit to generate output that is perceptible by the patient. Such output can be in the form of visible message, such as the light-up or blinking of a light emitting diode (LED) or the text message displayed in a liquid crystal display (LCD), or in the form of audible message such as beep, ringing tone, or pre-recorded voice messages played by a speaker, or in the form of discernible vibration by a vibrator. According to the patient's preference, one or multiple types of warning message can be respectively turned on or off. For example, the visible warning message can be turned on while the audible warning message can be turned off during the night if the patient chooses not to be disturbed during sleep even if the implant device detects AF. Besides generating warning messages, some diagnostic information that is received from the implant device and stored in memory (e.g., the heart rate) can also be provided to the patient in the form of visual or audible messages.

The external portable device, via its remote communication unit, can further communicate with the remote service center. Such long-range communication apparatus can be in the form of a mobile radio network, or a fixed-line telecommunication network, or the internet, as well known in the art. Examples of such long-range communication apparatus have been taught in US 6,470,215, US 6,574,509 and US 6,622,043, all are assigned to the assignee of the present invention.

In one typical embodiment, the external portable device transmits the implant device status information (e.g., battery status, sensing impedance, etc.) as well as relevant diagnostic information (e.g., AF burden, ectopic beat frequency, etc.) to the remote service center according to a predefined transmission frequency and schedule (e.g., every midnight, etc.). Yet in another typical embodiment, the external portable device communicates with the remote service center in a trigger mode, for example, upon receiving a warning signal from the implant device, or upon the patient trigger. In such cases, the external portable device transmits critical diagnostic information stored in device memory (e.g., AF burden, mean heart rate, the subcutaneous ECG snapshot, etc.) to the remote service center.

The remote service center receives the information via compatible communication protocols, then sends acknowledgment back to the external portable device, which may generate visible or audible output indicating receipt of the acknowledgment. The data received by the remote service center is stored in a central database, and is promptly presented to the patient's physician or responsible expert through proper means, such as fax, email, and text message as known in the art. By reviewing the received diagnostic information, the physician can evaluate the patient's condition and provide expert advice to the patient who wishes to contact the physician before taking any action in response to the warning signals generated by the external portable device.

The method and apparatus for noise detection from subcutaneous ECG recordings is disclosed hereinafter. It shall be understood that the same principles are also applicable to noise detection from electrocardiograms (ECG) in general and in particular from intracardiac electrograms recorded by implantable pacemakers or defibrillators, or surface electrocardiograms recorded using conventional ECG machines, bedside ECG monitors, Holter ECG devices, automatic external defibrillators, etc.

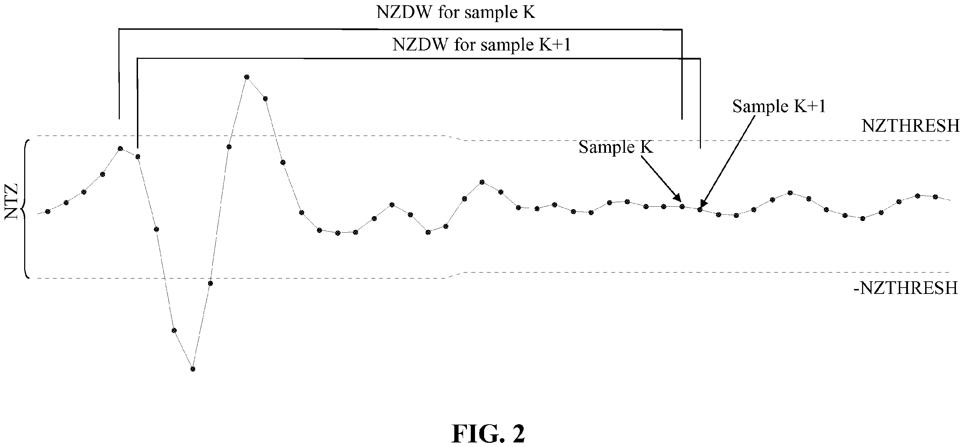

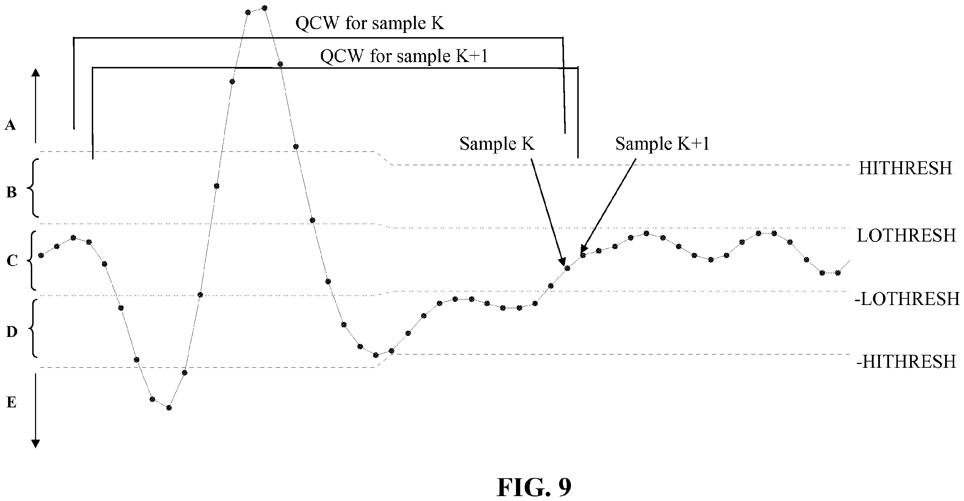

According to this invention, the noise condition is continuously evaluated on a sample-by-sample base. Figures 2 to 8 illustrate a first embodiment featuring only one upper and one lower threshold for a signal amplitude. Figures 9 to 14 illustrate a second embodiment featuring two upper and two lower thresholds for a signal amplitude.

As illustrated in Figures 2 and 9, each sample is preceded by a noise detection window (NZDW; see Figure 2) or a Quality Check Window (QCW; see figure 9), respectively, that has a user-programmable window length. In a typical embodiment, the NZDW is 125 ms, which corresponds to 32 samples at 256 Hz. In an alternative embodiment, the NZDW is 250 ms, which corresponds to 64 samples at 256 Hz. The noise detection window (NZDW) of the first embodiment is a quality check window in the broader sense of the claims.

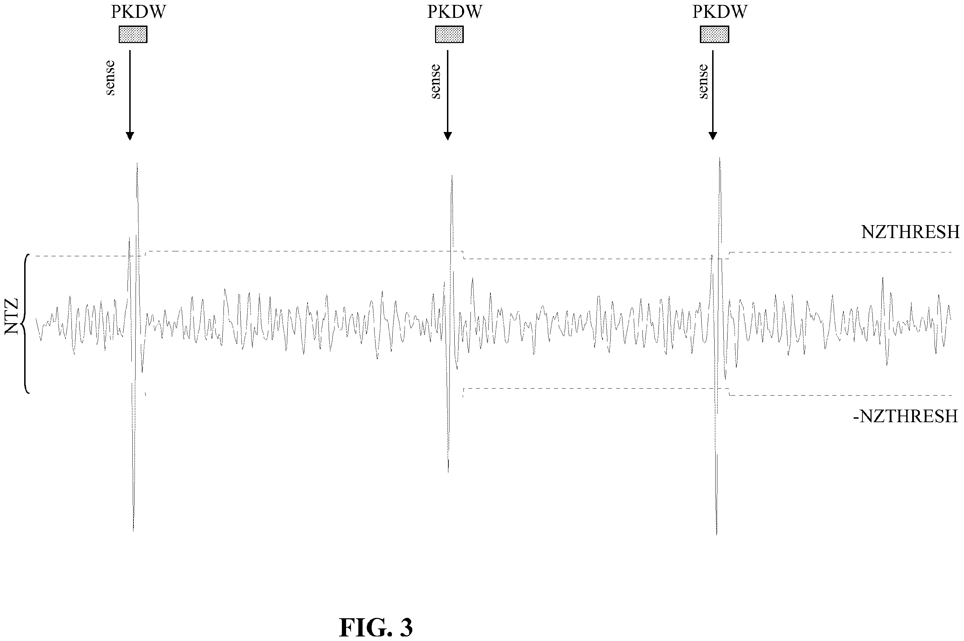

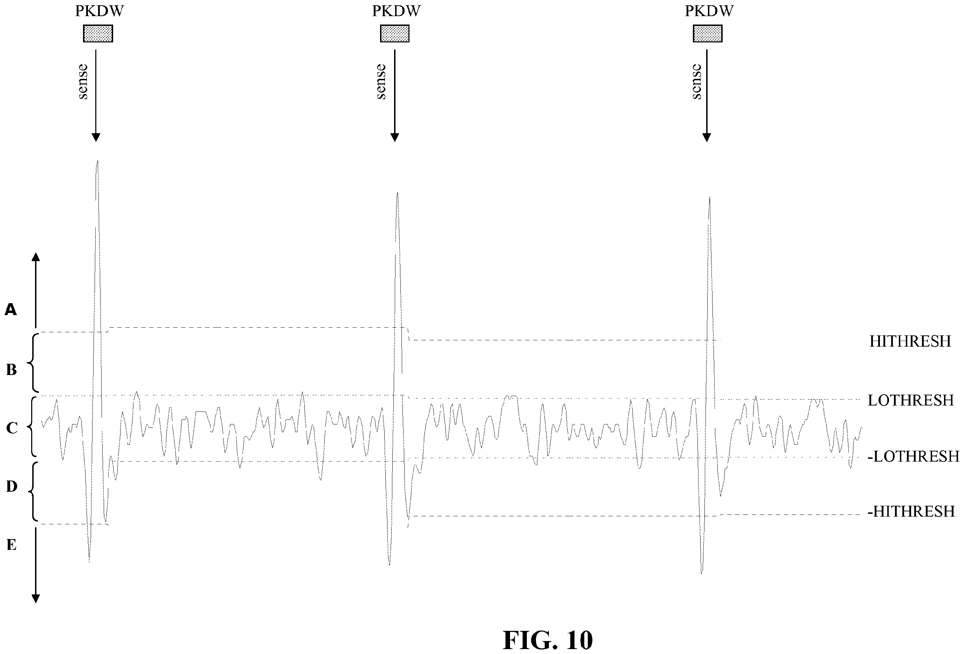

For recorded subcutaneous ECG signal, the device independently performs beat detection and generates sense markers. As illustrated in Figures 3 and 10, the device starts a peak detection window (PKDW) after each beat detection, triggered by the amplitude of a sample crossing a beat detection threshold, to search for the absolute peak amplitude (PEAK) of the signal. The PKDW spans from T1 before the sense marker to T2 after the sense marker. Both T1 and T2 are user-programmable parameters. In one typical embodiment, T1 is 40 ms and T2 is 60 ms. In another typical embodiment, T1 is 0 ms and T2 is 200 ms. Typically, the found PEAK value is the largest absolute signal amplitude of the QRS complex. Even if PKDW does not cover the whole QRS complex, the found PEAK value still represents the signal amplitude around the sense marker.

According to the first embodiment, the device maintains a (single) upper threshold called noise threshold (NZTHRESH) that is adapted to the signal amplitude. The device further maintains a (single) lower threshold having the same magnitude as the noise threshold (NZTHRESH) but the opposite sign and thus is called -NZTHRESH. In the following, it is assumed that the ECG signal has zero baseline after applying the front-end high-pass filter, thus the positive NZTHRESH and the negative -NZTHRESH form a noise tolerance zone (NTZ). Thus, the positive NZTHRESH and the negative -NZTHRESH define three amplidude ranges, namely a high amplitude range for amplitudes above NZTHRESH, a medium amplitude range corresponding to the noise tolerance zone (NTZ) and a low amplitude range for amplitudes below -NZTHRESH. During device initialization, the NZTHRESH is set to MIN_NZTHRESH, which is the user-programmable minimum noise threshold. For example, the MIN_NZTHRESH can be selected from a list of predefined voltage values such as 0.025 mV, 0.05 mV, 0.075 mV, 0.1 mV, etc. Then after detection of each beat, the NZTHRESH is automatically adapted to the found PEAK value as described in details below.

According to the second embodiment, the device maintains at least two upper thresholds or threshold vectors, a high threshold (HITHRESH) and a low threshold (LOTHRESH), both of which are adapted to the signal amplitude. Two negative lower thresholds with the same magnitudes -HITHRESH and -LOTHRESH are also provided. Typically, the magnitude of HITHRESH is larger than LOTHRESH. During device initialization, HITHRESH is set to MIN_HITHRESH, and LOTHRESH is set to MIN_LOTHRESH. Both MIN_HITHRESH and MIN_LOTHRESH are user-programmable, and they respectively represent the minimum thresholds that are allowed for HITHRESH and LOTHRESH. For example, the MIN_HITHRESH can be selected from a list of predefined voltage values such as 0.025 mV, 0.05 mV, 0.075 mV, 0.1 mV, etc, and the MIN_LOTHRESH can be defined as a fraction (e.g. 50%) of the MIN_HITHRESH.

In the following discussions, it is assumed that the ECG signal has zero baseline after applying the front-end high-pass filter, thus HITHRESH and -HITHRESH are symmetric around the baseline, and LOTHRESH and -LOTHRESH are also symmetric around the baseline. According to a typical embodiment of this invention, HITHRESH and LOTHRESH divide the signal space into five non-overlapping amplitude ranges, that in the following are called subspaces: A is the subspace (amplitude range) above HITHRESH, B is the subspace (amplitude range) between LOTHRESH and HITHRESH, C is the subspace (amplitude range) between -LOTHRESH and LOTHRESH, D is the subspace (amplitude range) between -LOTHRESH and -HITHRESH, and E is the subspace (amplitude range) below -HITHRESH. Obviously, if the baseline of ECG signal is not zero, the threshold vectors can be defined in an asymmetric manner to ensure subspace C encompasses the baseline.

After each beat detection, NZTHRESH or both HITHRESH and LOTHRESH are automatically adapted to the found PEAK value as described in details below.

As illustrated in Figure 3, after detection of each beat, the NZTHRESH remains unchanged during the PKDW. Immediately after the PKDW, the NZTHRESH is recalculated based on the previous NZTHRESH value, the present PEAK value (denoted as NEW_PEAK), and the smoothed PEAK amplitudes of the past beats (denoted as OLD_PEAK). Specifically, NZTHRESH is calculated in 3 steps:

$$NEW\_PEAK = min(NEW\_PEAK, p*OLD\_PEAK); \qquad (1.1)$$

$$OLD\_PEAK = OLD\_PEAK*c1 + NEW\_PEAK*c2; \qquad (1.2)$$

$$NZTHRESH = max(MIN\_NZTHRESH, OLD\_PEAK*f); \qquad (1.3)$$

Similarly, as illustrated in FIG. 10, after each beat detection, the HITHRESH and LOTHRESH remain unchanged during the PKDW. Immediately after the PKDW, the HITHRESH is recalculated based on the previous HITHRESH value, the present PEAK value (denoted as NEW_PEAK), and the smoothed PEAK amplitudes of the past beats (denoted as OLD PEAK). Similarly, the LOTHRESH is recalculated based on the previous LOTHRESH value, the present PEAK value (denoted as NEW_PEAK), and the smoothed PEAK amplitudes of the past beats (denoted as OLD_PEAK).

Specifically, HITHRESH and LOTHRESH are calculated in following steps:

$$NEW\_PEAK = min(NEW\_PEAK, p*OLD\_PEAK); \qquad (2.1)$$

$$OLD\_PEAK = OLD\_PEAK*c1 + NEW\_PEAK*c2; \qquad (2.2)$$

$$HITHRESH = max(MIN\_HITHRESH, OLD\_PEAK*fh); \qquad (2.3)$$

$$LOTHRESH = max(MIN\_LOTHRESH, OLD\_PEAK*fl); \qquad (2.4)$$

In Equations (1.1) and (2.1), p is a user-programmable parameter that is greater than 1.0. In a preferred embodiment, p is set to 1.25 or 1.5. Therefore, the NEW_PEAK is limited to the minimum of PEAK amplitude measured for the current beat and the OLD_PEAK amplitude scaled by the coefficient p. This prevents the abrupt increase of HITHRESH or LOTHRESH due to the detection of a very large PEAK amplitude (e.g. due to impulse noise).

In Equations (1.2) and (2.2), c1 and c2 are also user-programmable weighting coefficients that are between 0 and 1, and c1+c2=1.0. In a preferred embodiment, c1=0.5 and c2=0.5. Alternative settings can also be used, for example, c1=0.75 and c2=0.25, or c1=0.25 and c2=0.75, etc. This equation calculates the weighted average of the OLD_PEAK and the NEW_PEAK, and the result is assigned to OLD PEAK. Therefore, OLD PEAK represents the smoothed PEAK amplitudes of the past beats. The updated OLD_PEAK is then maintained until the next beat detection.

In Equations (1.3), (2.3) and (2.4), f or fh, respectively, and fl are also user-programmable parameters that are less than 1.0. In a preferred embodiment, for fh is set to 0.375 for calculating HITRRESH, and fl is set to 0.125 for calculating LOTHRESH. Other exemplary settings can also be used, for example fh=0.5, fl=0.25, etc. Therefore, equation (1.3) or (2.3) sets NZTHRESH or HITHRESH, respectively, to a predefined percentage of the updated OLD_PEAK with the lower limit of the predefined MIN_NZTHRESH or MIN_HITHRESH, respectively, while equation (2.4) sets LOTHRESH to another predefined percentage of the updated OLD_PEAK with the lower limit of the predefined MIN_LOTHRESH.

Because the OLD_PEAK represents the weighted average of the signal peak amplitude, the calculated NZTHRESH represents a tolerance level for noise. In other words, for relatively clean ECG signal with high SNR, most samples between adjacent QRS complexes are expected to be bounded by ±NZTHRESH and thus fall into the medium amplitude range as used according to the first embodiment. On the other hand, for relatively noisy ECG signal with low SNR, many samples between adjacent QRS complexes are expected to fall outside the NTZ, that is, outside the medium amplitude range.

Similarly, because the OLD_PEAK represents the weighted average of the signal peak amplitude, the calculated HITHRESH and LOTHRESH represent two different tolerance levels for noise. In other words, for relatively clean ECG signal with high SNR, most samples between adjacent QRS complexes are expected to fall in the subspace C, that is the medium amplitude range according to the second embodiment. On the other hand, when noise is present and SNR is low, many samples between adjacent QRS complexes are expected to fall in the subspaces B and D, and even in the subspaces A and E.

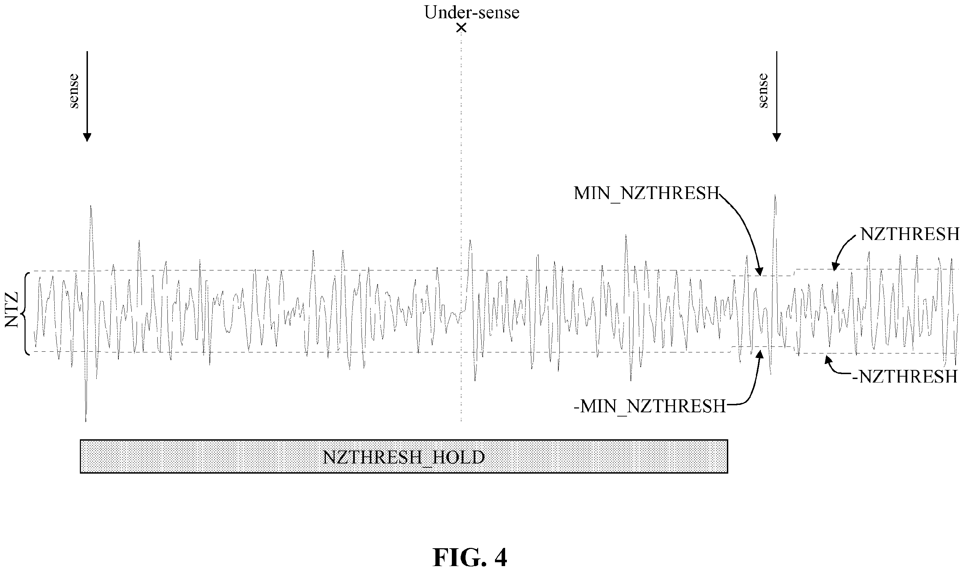

According to the first embodiment, either the NZTHRESH is kept constant until the next beat detection when it is again updated based on equations (1)-(3), or NZTHRESH is lowered to MIN_NZTHRESH when no beat is detected after a user-programmable duration (NZTHRESH_HOLD). In a preferred embodiment, NZTHRESH_HOLD is set to 2 seconds. Alternative settings can also be used, for example, NZTHRESH_HOLD can be set to 1 second, 2.5 seconds, etc. As illustrated in Figure 4, device sometimes may under-sense the QRS complex (marked by the vertical dashed line and a cross). In such cases, NZTHRESH may not be properly adapted to the signal amplitude, thus may be too high to detect noise. Therefore, by lowering the NZTHRESH to MIN_NZTHRESH after NZTHRESH HOLD duration, device can become more alert to noise condition.

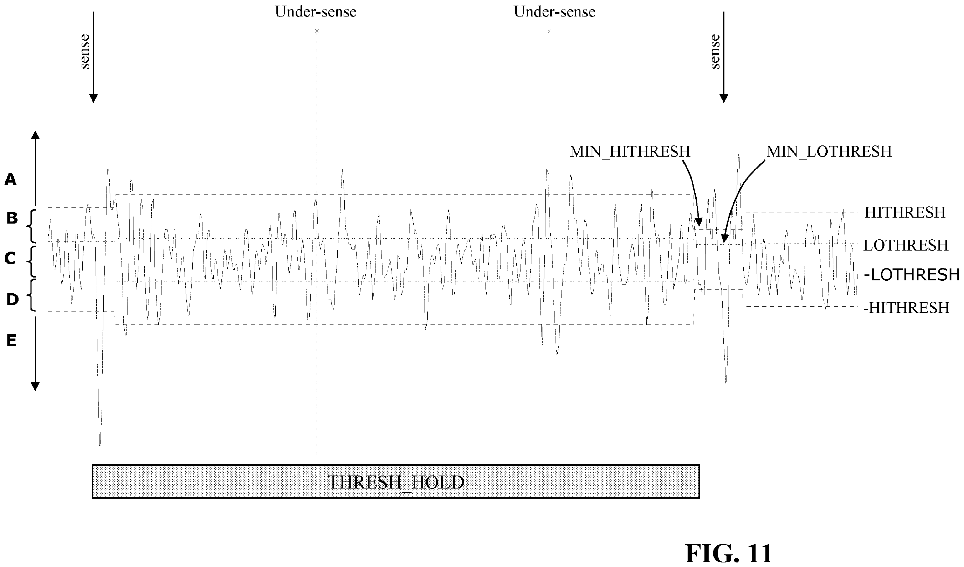

Similarly, according to the second embodiment, either the HITHRESH and LOTHRESH are kept constant until the next beat detection when it is again updated based on equations (2.1) to (2.4), or they are lowered to their respective minimum threshold values when no beat is detected after a user-programmable duration (THRESH_HOLD). In a preferred embodiment, THRESH_HOLD is set to 2 seconds. Alternative settings can also be used, for example, THRESH_HOLD can be set to 1 second, 2.5 seconds, etc. As illustrated in FIG. 11, device sometimes may under-sense the QRS complexes (marked by the vertical dashed lines). In such cases, HITHRESH and LOTHRESH may not be properly adapted to the signal amplitude. Therefore, by lowering HITHRESH to MIN_HITHRESH and LOTHRESH to MIN_LOTHRESH after THRESH_HOLD duration, device can become more alert to noise condition.

Now, noise condition evaluation or signal quality evaluation, respectively, is described.

First, noise condition evaluation according to the first embodiment is described.

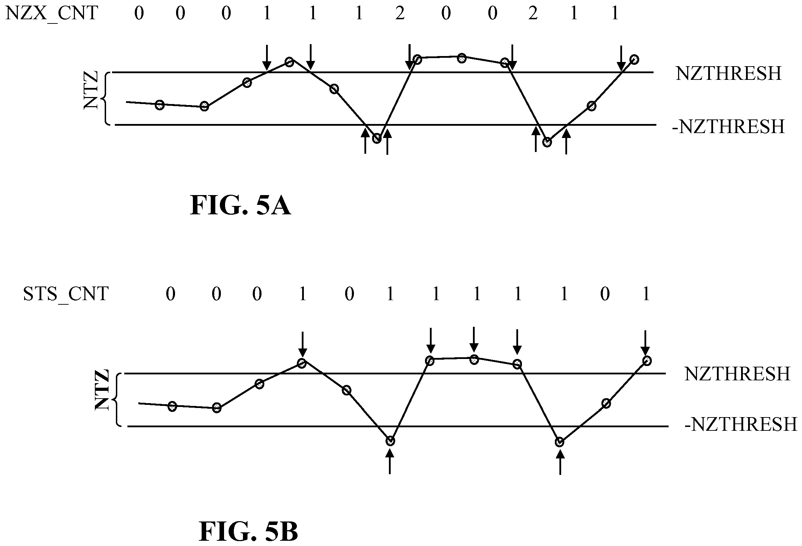

For each NZDW, noise condition is evaluated by two metrics: the noise threshold crossing count (NZX_CNT), and the supra-threshold sample count (STS_CNT). Figure 5A illustrates the method of counting noise threshold crossings (each arrow points to a noise threshold crossing). Figure 5B illustrates the method of counting supra-threshold samples (each arrow points to a supra-threshold sample).

Assume the current sample amplitude is $X(k)$, and the previous sample amplitude is $X(k-1)$. The corresponding noise thresholds for the two samples are $NZTHRESH(k)$ and $NZTHRESH(k-1)$, respectively. The STS_CNT for the k-th sample is set to 1 if

$X(k) \geq NZTHRESH(k)$ OR $X(k) \leq -NZTHRESH(k)$

Otherwise, i.e. $-NZTHRESH(k) < X(k) < NZTHRESH(k)$, the STS_CNT for the k-th sample is set to 0.

The NZX_CNT for the k-th sample is set to 1 if

$X(k) \geq NZTHRESH(k)$ OR $X(k) \leq -NZTHRESH(k)$

AND

$-NZTHRESH(k-1) < X(k-1) < NZTHRESH(k-1)$

On the other hand, the NZX_CNT for the k-th sample is set to 2 if

$X(k) \geq NZTHRESH(k)$ AND $X(k-1) \leq -NZTHRESH(k-1)$

OR

$X(k) \leq -NZTHRESH(k)$ AND $X(k-1) \geq NZTHRESH(k-1)$

Otherwise, i.e. both $X(k)$ and $X(k-1)$ are within the NTZ or both are $\geq$ NZTHRESH or both are $\leq$ -NZTHRESH, the NZX_CNT for the k-th sample is set to 0.

According to the first embodiment, both NZX_CNT and STS_CNT are continuously assessed for each sample. Specifically, the device maintains two first-in-first-out (FIFO) buffers that respectively store the NZX_CNT and STS_CNT for all samples within the NZDW. The sum of NZX_CNT (denoted as NZX_SUM) and the sum of STS_CNT (denoted as STS_SUM) for all samples within the NZDW are respectively calculated. The noise condition for the specific NZDW is indicated when any one of the following three conditions is met:

(1) NZX_SUM exceeds a first user-programmed threshold (TX1); OR

(2) STS_SUM exceeds a second user-programmed threshold (ST1); OR

(3) NZX_SUM exceeds a third user-programmed threshold (TX2) AND STS_SUM exceeds a fourth user-programmed threshold (ST2).

Preferably, the thresholds are set so that $TX2 \leq TX1$ and $ST2 \leq ST1$. Assume the NZDW has 32 samples with sampling frequency 256 Hz (i.e. NZDW has duration of 125 ms), a typical set of thresholds are TX1 = 10, TX2 = 25, TX3 = 8, and TX4 = 8. Condition (1) and (3) are mainly useful to detect high frequency noises, whereas the condition (2) is mainly useful to detect saturation noise.

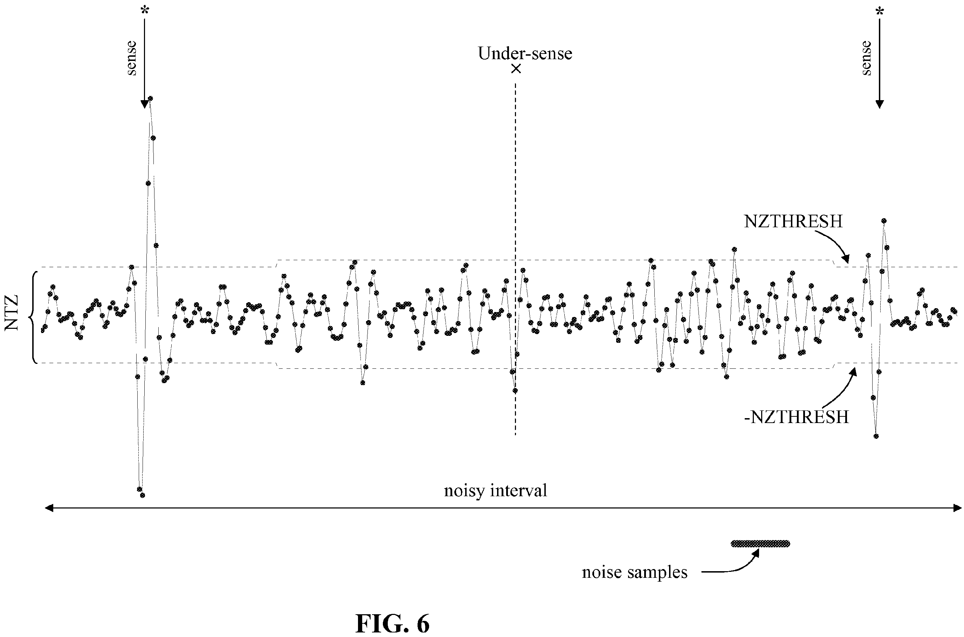

Refer to Figure 6. In a preferred embodiment, when noise condition is indicated for any NZDW associated with a specific sample, this sample is considered as a noise sample. Then the detected beats immediately before and immediately after this noise sample are labeled as unreliable detections (marked by * in Figure 6), and the interval between this pair of unreliable detections is labeled as noisy interval. In addition, the interval before the first unreliable detection and the interval after the second unreliable detection can also be labeled as noisy intervals.

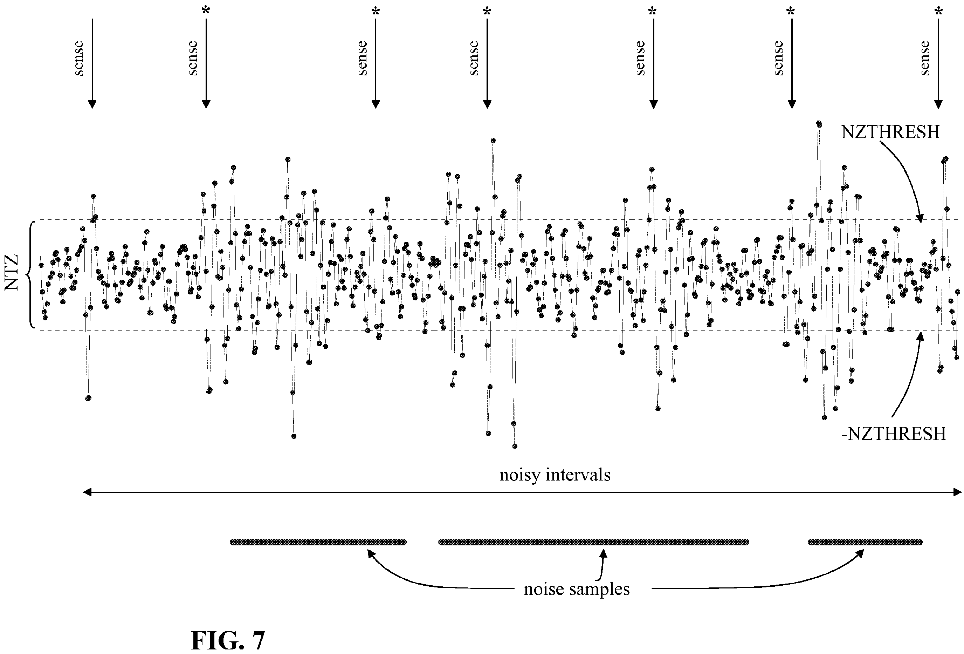

Refer to Figure 7. In another embodiment, noise detection is evaluated on a moving window basis. The moving window consists of Y samples. When X out of Y samples are determined as noise samples, the noise condition is confirmed. For example, assume Y=64 and X=32, then noise condition is indicated when more than half of the samples in the moving window are noisy samples. Then the detected beats immediately before and immediately after this window are labeled as unreliable detections (marked by * in Figure 7), and the intervals associated with any pair of unreliable detections are labeled as noisy interval.

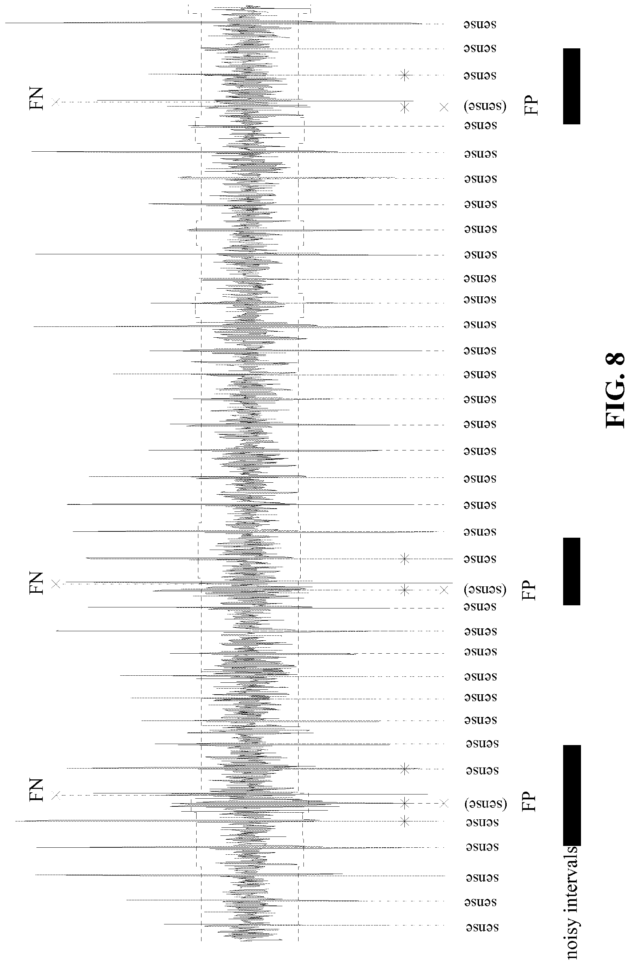

Figure 8 shows an example of ECG noise detection result. In this example, beat detections are marked by downward dashed lines and labeled by "sense" markers. False positive (FP) detections occur 3 times (indicated by "sense" in parenthesis and "X" marker at the end of the downward dashed line), and false negative (FN, or under-sensing) occurs in 3 beats (marked by upward dashed lines ending with the "X" marker). According to the noise detection algorithm described above, 7 senses are determined as unreliable detections (marked by * on the downward dashed lines), and the intervals associated with these unreliable detections are labeled as noisy intervals. These unreliable senses and the associated noisy intervals are preferably excluded from rate and rhythm analysis, therefore improving the diagnostic accuracy of the device.

Now, signal quality evaluation according to the second embodiment is described.

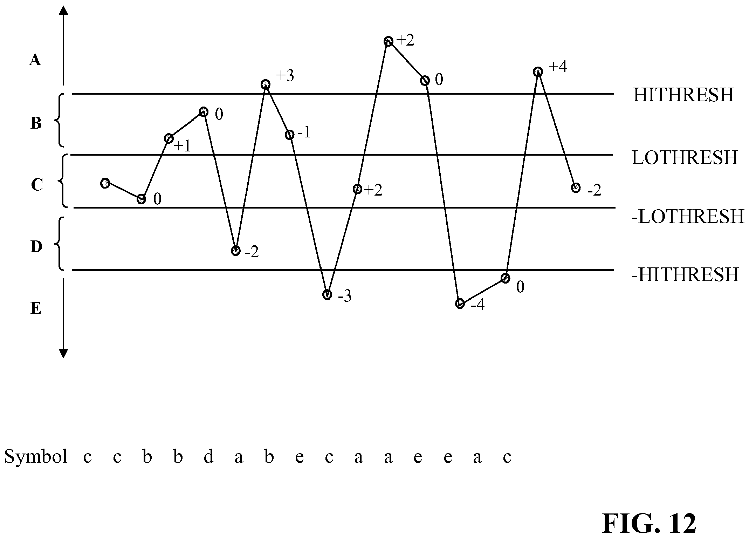

For each QCW, signal quality is evaluated by means of a symbolic dynamics approach. FIG. 12 illustrates the basic concept of converting the signal samples to symbols, and then extracting the symbolic dynamics.

First, each signal sample is converted to one of five symbols according to which subspace (amplitude range) it belongs to. In other words, a sample is assigned a symbol 'a' if it falls in subspace A, or 'b' if it falls in subspace B, or 'c' if it falls in subspace C, or 'd' if it falls in subspace D, or 'e' if it falls in subspace E. Therefore, in FIG. 12, the 15 signal samples are converted to a string of 15 symbols: c-c-b-b-d-a-b-e-c-a-a-e-e-a-c.

Then for any two adjacent sample pairs, their difference is coded based on the symbol change step (SCS). The symbol change step (SCS) is a threshold crossing number reflecting the number of thresholds between each two adjacent signal samples of each sample pair. If two adjacent signal samples forming a sample pair fall into the same amplitude range, there is no threshold between these sample and thus no symbol change, e.g. 'a-a', then the threshold crossing number SCS is set to 0. If the symbol change is one step, e.g., 'a-b', that is, if there is one threshold between the adjacent signal sample of a sample pair, then the threshold crossing number SCS is set to 1. If the symbol change is two steps, e.g., 'a-c', that is, if there are two thresholds between the adjacent signal sample of a sample pair, then the threshold crossing number SCS is set to 2. If the symbol change is three steps, e.g. 'a-d', that is, if there are three thresholds between the adjacent signal sample of a sample pair, then the threshold crossing number SCS is set to 3. If the symbol change is four steps, e.g., 'a-e', that is, if there are for thresholds between the adjacent signal sample of a sample pair, then the threshold crossing number SCS is set to 4. Table 1 below lists all possible symbol pairs and their respective SCS codes. The SCS sign indicates the direction of amplitude change, thus a positive SCS indicates amplitude increase across at least one threshold between the sample pair, and negative SCS indicates amplitude decrease across at least on threshold between the sample pair. Therefore, in FIG. 12, the 14 sample pairs (excluding the first sample) can be translated to a set of SCS codes: {0, +1, 0, -2, +3, -1, -3, +2, +2, 0, -4, 0, +4, -2}, each SCS code reflecting a threshold crossing number. The sequence of SCS codes (threshold crossing numbers) eliminate the detailed signal amplitude information, but keep the robust properties of the signal dynamics by a coarse-graining of the measurements.

Table 1. Symbol pairs and the corresponding SCS codes

| scs | 0 | +1 | +2 | +3 | +4 | -1 | -2 | -3 | -4 |
|---|---|---|---|---|---|---|---|---|---|
| Symbol pairs | a-a | b-a | c-a | d-a | e-a | a-b | a-c | a-d | a-e |
| | b-b | c-b | d-b | e-b | | b-c | b-d | b-e | |
| | c-c | d-c | e-c | | | c-d | c-e | | |
| | d-d | e-d | | | | d-e | | | |
| | e-e | | | | | | | | |

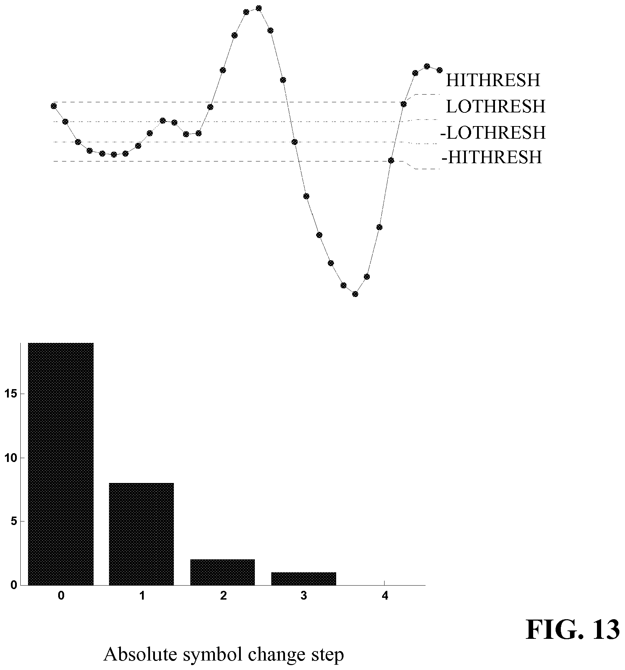

For each QCW, the corresponding sequence of SCS codes (threshold crossing numbers) is then analyzed. According to a typical embodiment, a histogram of absolute SCS codes is generated. FIG. 13 illustrates one example in which 32 sample pairs result in a distribution of absolute SCS. In this example, 21 sample pairs have no symbol change (SCS=0), 8 sample pairs have one-step symbol change (SCS=+1 or -1), 2 sample pairs have two-step symbol change (SCS=+2 or -2), and 1 sample pair has three-step symbol change (SCS=+3 or -3). No sample pair has four-step symbol change (SCS=+4 or -4).

Next, a complexity index (CI) is calculated to quantify the signal quality for the QCW. Higher CI indicates lower signal quality, whereas lower CI indicates higher signal quality. According to a typical embodiment of the present invention, the CI is calculated as the weighted sum of the absolute SCS counts:

$$CI = w1*SCS1 + w2*SCS2 + w3*SCS3 + w4*SCS4 \qquad (5)$$

Here SCS1 is the total count of SCS=1 and SCS=-1, SCS2 is the total count of SCS=+2 and SCS=-2, SCS3 is the total count of SCS=+3 and SCS=-3, and SCS4 is the total count of SCS=+4 and SCS=-4. Coefficients w1, w2, w3, and w4 are predefined values that assign proper weights to different symbol change steps. In a typical example, w1=1, w2=4, w3=8, and w4=16.

According to this invention, the CI is continuously evaluated to monitor the signal quality. Specifically, after receiving each signal sample, the device examines the past samples in the associated QCW. All samples in the QCW are converted to symbols and the symbolic dynamics are represented by a sequence of SCS codes. Then the histogram of absolute SCS is constructed and the corresponding CI is calculated. Higher CI indicates lower signal quality, whereas lower CI indicates higher signal quality. According to a typical embodiment, the sample-by-sample CI is compared to a predefined CI threshold to determine whether the ECG signal has sufficient quality for reliable cardiac beat detection.

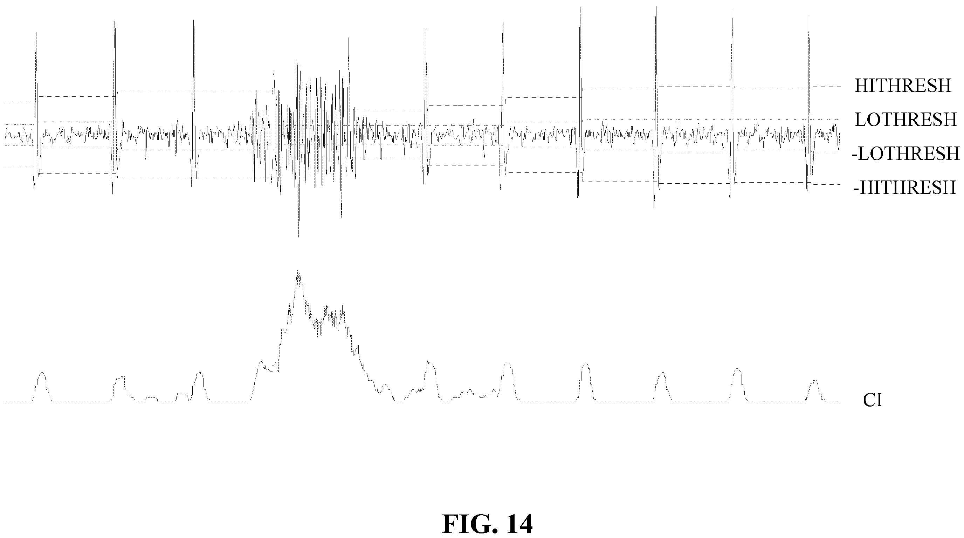

FIG. 14 shows an example where a segment of subcutaneous ECG is shown in the top trace (together with the adaptive threshold vectors), and the calculated CI is plotted in the bottom trace. Clearly, the CI remains small for most cardiac cycles, with only slight increase during the QRS complexes. In contrast, the burst of noise (after the third QRS complex) is associated with sharp increase of the CI.

Finally, it should be understood that the above description is merely a representative embodiment of the present invention, and other variants of the method with the same principle are within the scope of this invention. For example, the signal samples can be converted to different sets of symbols (i.e. not limited to 5 symbols) by using multiple threshold vectors. Also, the symbolic dynamics can be coded by grouping multiple symbols (e.g., code three adjacent symbols instead of two adjacent symbols).

**Claims**

1. A computer-implemented method for determination of noise in a sampled cardiac electrical signal, said sampled cardiac electrical signal comprising a consecutive sequence of samples, each sample having an amplitude, the amplitudes in general being positive or negative, the method comprising the steps of

   - providing a signal quality check window that comprises a number of consecutive samples
   - providing at least one upper threshold for a signal amplitude
   - providing at least one lower threshold for a signal amplitude said at least one upper threshold and said at least one lower threshold defining at least three amplitude ranges, a high range for signal amplitude exceeding said upper threshold, a medium range for amplitudes between said upper threshold and said lower threshold and a low range for amplitude below said lower threshold
   - comparing each sample within said quality check window with said upper threshold and with said lower threshold; and
   - performing a signal quality analysis for said signal sample by: a) analyzing the changes in amplitude of successive samples within said quality check window, said change in amplitude being determined for any two adjacent sample pairs within said quality check window by comparing to which amplitude ranges the amplitudes of the signal samples of the respective sample pair belong to, and by b) determining a threshold crossing number reflecting the number of thresholds between each two adjacent signal samples of each sample pair.

2. The method according to claim 1, comprising determining a noise threshold crossing sum of said threshold crossing numbers for a respective quality check window.

3. The method according to claim 2, wherein the noise threshold crossing sum is a weighted sum with predetermined weights for each threshold crossing number magnitude.

4. The method according to claim 1, wherein the cardiac electrical signal is an electrocardiogram (ECG) signal a surface electrocardiogram signal, a subcutaneous electrocardiogram signal, or a intracardiac electrogram signal.

5. The method according to claim 1, wherein the magnitudes of the at least one upper threshold (NZTHRESH; HITHRESH, LOTHRESH) and the at least one lower threshold (-NZTHRESH; -HITHRESH, -LOTHRESH) are identical; and/or the upper noise threshold (NZTHRESH; HITHRESH, LOTHRESH) is positive and the lower noise threshold (-NZTHRESH; -HITHRESH, -LOTHRESH) is negative.

6. The method according to claim 1, wherein an ECG beat detection is performed by determining if an amplitude of any sample exceeds a beat detection threshold, and generating a sense marker at this sample and wherein a peak detection window (PKDW) around the sense marker, having predefined length and position with respect to the sense marker, is defined, and wherein a maximum absolute amplitude of all samples in the peak detection window (PKDW) is determined as a peak value (PEAK).

7. The method according to claim 1, wherein the at least one upper threshold (NZTHRESH; HITHRESH, LOWTHRESH) and/or the lower noise threshold (-NZTHRESH; -HITHRESH,-LOTHRESH) are recalculated after each ECG beat detection

8. The method according to claim 7, wherein the at least one upper noise threshold (NZTHRESH, HITHRESH) is recalculated after an ECG beat detection, each ECG beat detection yielding a peak value (PEAK) corresponding to a maximum absolute amplitude measured during a peak detection window (PKDW), by performing the following steps:

(a) calculating a minimum value (NEW PEAK) by taking the minimum of a peak value (PEAK) of a recently performed ECG beat detection, and a weighted average value (OLD_PEAK) calculated directly after the previous ECG beat detection and being scaled by a first predefined scaling factor (p) larger than one,
(b) calculating a weighted average value (OLD PEAK) of the minimum value (NEW_PEAK) calculated in step (a), and the weighted average value (OLD PEAK) calculated directly after the previous ECG beat detection, the minimum value (NEW_PEAK) and the weighted average value (OLD PEAK) each being weighted by respective predefined weighting factors (w1, w2), the predefined weighting factors (w1, w2) summing to one, and
(c) recalculating the upper noise threshold (NZTHRESH) by taking the maximum of either a predefined minimum threshold value (MIN_NZTHRESH), or the weighted average value (OLD PEAK) calculated in step (b) and being scaled by a second predefined scaling factor (f), the second predefined scaling factor (f) being larger than zero and smaller than one.

9. The method according to claim 1, wherein ECG peak detections immediately before and immediately after a sample for which a low signal quality was indicated, are labeled as unreliable.

10. The method according to claim 1, wherein the noise conditions of samples are evaluated on a moving window basis such that if the number of samples in any window containing a predefined number (Y) of samples, for which a low signal quality is determined, exceeds another predefined number (X), ECG beat detections immediately before and after the window are labeled as unreliable.

11. The method according to claim 2, said method comprising the steps of

- providing a sample number (NZDW), an upper noise threshold (NZTHRESH) as upper threshold and a lower noise threshold (-NZTHRESH) as lower threshold,
- defining a quality check window comprising a number (NZDW) of samples including a last sample (k), said number corresponding to said sample number,
- determining a supra-threshold sample sum (STS_SUM) corresponding to the number of samples in said quality check window that have an amplitude that either exceeds said upper noise threshold (NZTHRESH) or falls below said lower noise threshold (-NZTHRESH), respectively,
- determining a noise threshold crossing sum (NZX_SUM) corresponding to the sum of the threshold crossing number (NZX CNT) of noise threshold-crossings between each two consecutive samples in the quality check window,
- indicating a low signal quality for each last sample (k) of each noise detection window, if

- said noise threshold crossing sum (NZX_SUM) exceeds a first predefined value (TX1), or
- said supra-threshold sample sum (STS_SUM) exceeds a second predefined value (ST1), or
- said noise threshold crossing sum (NZX_SUM) exceeds a third predefined value (TX2) and said supra-threshold sample sum (STS_SUM) exceeds a fourth predefined value (ST2).

12. The method of claim 3, wherein the noise threshold crossing sum is a complexity index, said method further comprising the steps of
comparing said complexity index with a complexity index threshold and
generating a low signal quality indicating signal if said complexity index exceeds said complexity index threshold.

13. A medical device having means for processing a sampled cardiac electrical signal and a processing unit, said cardiac electrical signal comprising a consecutive sequence of samples, each sample having an amplitude, the amplitudes in general being positive or negative, wherein the medical device comprises a memory, said memory comprising data defining a signal quality check window that comprises a number of consecutive samples, at least one upper threshold for a signal amplitude and at least one lower threshold for a signal amplitude, said at least one upper threshold and said at least one lower threshold defining at least three amplitude ranges, a high range for signal amplitude exceeding said upper threshold, a medium range for amplitudes between said upper threshold and said lower threshold and a low range for amplitude below said lower threshold, wherein said processing unit is configured

- to compare each sample within said quality check window with said upper threshold and with said lower threshold; and
- to perform a signal quality analysis for said signal sample by: a) analyzing the changes in amplitude of successive samples within said quality check window, said change in amplitude being determined for any two adjacent sample pairs within said quality check window by comparing to which amplitude ranges the amplitudes of the signal samples of the respective sample pair belong to, and by b) determining a threshold crossing number reflecting the number of thresholds between each two adjacent signal samples of each sample pair.

14. The medical device according to claim 13, wherein the processing unit is further configured to perform an ECG beat detection by determining if an amplitude of any sample exceeds a beat detection threshold, and generating a sense marker at this sample, and to determine a peak value (PEAK) associated with each ECG peak detection, by defining a peak detection window (PKDW) around the sense marker, having predefined length and position with respect to the sense marker, and determining a maximum absolute amplitude of all samples in the peak detection window (PKDW) as a peak value (PEAK).

15. The medical device according to claim 13, wherein the processing unit is further configured to recalculate the at least one upper threshold (NZTHRESH; HITHRESH, LOTHRESH) after an ECG beat detection, each ECG beat detection yielding a peak value (PEAK) corresponding to a maximum absolute amplitude measured during a peak detection window (PKDW), by performing the following steps:

(a) calculating a minimum value (NEW PEAK) by taking the minimum of a peak value (PEAK) of a recently performed ECG beat detection, and a weighted average value (OLD PEAK) calculated directly after the previous ECG beat detection and being scaled by a first predefined scaling factor (p) larger than one,
(b) calculating a weighted average value (OLD_PEAK) of the minimum value (NEW_PEAK) calculated in step (a), and the weighted average value (OLD PEAK) calculated directly after the previous ECG beat detection, the minimum value (NEW_PEAK) and the weighted average value (OLD PEAK) each being weighted by respective predefined weighting factors (w1, w2), the predefined weighting factors (w1, w2) summing to one, and
(c) recalculating the at least one upper noise threshold (NZTHRESH; HITHRESH, LOTHRESH) by taking the maximum of either a predefined minimum threshold value (MIN NZTHRESH; MIN_HITHRESH, MIN_LOTHRESH), or the weighted average value (OLD PEAK) calculated in step (b) and being scaled by a second predefined scaling factor (f; fh, fl), the second predefined scaling factor (f; fh, fl) being larger than zero and smaller than one.

16. The medical device according to claim 13, wherein the processing unit is further configured to evaluate the signal quality of signal samples on a moving window basis such that if the number of samples in any quality check window containing a predefined number (Y) of samples, for which a low signal quality is determined, exceeds another predefined number (X), ECG beat detections immediately before and after the window are labeled as unreliable.

**Patentansprüche**

1. Computerimplementiertes Verfahren zum Bestimmen von Rauschen in einem abgetasteten kardialen elektrischen Signal, wobei das genannte abgetastete kardiale elektrische Signal eine aufeinanderfolgende Folge von Abtastungen umfasst, wobei jede Abtastung eine Amplitude hat, wobei die Amplituden allgemein positiv oder negativ sind, wobei das Verfahren die folgenden Schritte umfasst

- Bereitstellen eines Signalqualitätskontrollfensters, das eine Anzahl aufeinanderfolgender Abtastungen umfasst,
- Bereitstellen wenigstens einer oberen Schwelle für eine Signalamplitude,
- Bereitstellen wenigstens einer unteren Schwelle für eine Signalamplitude, wobei die genannte wenigstens eine obere Schwelle und die genannte wenigstens eine untere Schwelle wenigstens drei Amplitudenbereiche definieren, einen hohen Bereich für Signalamplituden, die die genannte obere Schwelle übersteigen, einen mittleren Bereich für Amplituden zwischen der genannten oberen Schwelle und der genannten unteren Schwelle und einen unteren Bereich für Amplituden unter der genannten unteren Schwelle,

- Vergleichen jeder Abtastung innerhalb des genannten Qualitätskontrollfensters mit der genannten oberen Schwelle und mit der genannten unteren Schwelle; und

- Durchführen einer Signalqualitätsanalyse für die genannte Signalabtastung durch: a) Analysieren der Amplitudenänderungen aufeinanderfolgender Abtastungen innerhalb des genannten Qualitätskontrollfensters, wobei die genannte Amplitudenänderung für beliebige zwei benachbarte Abtastungspaare innerhalb des genannten Qualitätskontrollfensters bestimmt wird durch Vergleichen, zu welchen Amplitudenbereichen die Amplituden der Signalabtastungen des jeweiligen Abtastungspaares gehören, und b) durch Bestimmen einer Schwellenübergangszahl, die die Anzahl von Schwellen zwischen beliebigen zwei benachbarten Signalabtastungen jedes Abtastungspaares reflektieren.

2. Verfahren nach Anspruch 1, das das Bestimmen einer Rauschschwellenübergangssumme der genannten Schwellenübergangszahlen für ein jeweiliges Qualitätskontrollfenster aufweist.

3. Verfahren nach Anspruch 2, wobei die Rauschschwellenübergangssumme eine gewichtete Summe mit vorbestimmten Gewichten für jede Schwellenübergangszahlgröße ist.

4. Verfahren nach Anspruch 1, wobei das kardiale elektrische Signal ein Elektrokardiogramm- (EKG) -signal, ein Oberflächen-Elektrokardiogrammsignal, ein Signal eines subkutanen Elektrokardiogramms oder ein intrakardiales Elektrokardiodiagrammsignal ist.

5. Verfahren nach Anspruch 1, wobei die Größen der wenigstens einen oberen Schwelle (NZTHRESH; HITHRESH, LOTHRESH) und der wenigstens einen unteren Schwelle (-NZTHRESH; -HITHRESH, -LOTHRESH) identisch sind und/oder wobei die obere Rauschschwelle (NZTHRESH; HITHRESH, LOTHRESH) positiv ist und die untere Rauschschwelle (-NZTHRESH; -HITHRESH, -LOTHRESH) negativ ist.

6. Verfahren nach Anspruch 1, wobei eine EKG-Herzschlagdetektion durchgeführt wird durch Bestimmen, ob eine Amplitude einer beliebigen Abtastung eine Herzschlagdetektionsschwelle übersteigt, und Erzeugen eines Sense-Markers an dieser Abtastung und wobei ein Spitzendetektionsfenster (PKDW) um den Sense-Marker, das eine vordefinierten Länge und Position in Bezug auf den Sense-Marker hat, definiert wird und wobei eine maximale absolute Amplitude aller Abtastungen im Spitzendetektionsfenster (PKDW) als ein Spitzenwert (PEAK) bestimmt wird.

7. Verfahren nach Anspruch 1, wobei die wenigstens eine obere Schwelle (NZTHRESH; HITHRESH, LOTHRESH) und/oder die untere Rauschschwelle (-NZTHRESH; -HITHRESH, -LOTHRESH) nach jeder EKG-Herzschlagdetektion neuberechnet wird.

8. Verfahren nach Anspruch 7, wobei die wenigstens eine obere Rauschschwelle (NZTHRESH, HITHRESH) nach einer EKG-Herzschlagdetektion, wobei jede EKG-Herzschlagdetektion einen Spitzenwert (PEAK) ergibt, der einer während eines Spitzendetektionsfensters (PKDW) gemessenen maximalen absoluten Amplitude entspricht, durch Durchführen der folgenden Schritte neuberechnet wird:

(a) Berechnen eines Mindestwerts (NEW_PEAK) durch Nehmen des Minimums eines Spitzenwerts (PEAK) einer kürzlich durchgeführten EKG-Herzschlagdetektion und eines gewichteten Durchschnittswerts (OLD_PEAK), der unmittelbar nach der vorhergehenden EKG-Herzschlagdetektion berechnet und um einen ersten vordefinierten Skalierungsfaktor (p), der größer als eins ist, skaliert wird;
(b) Berechnen eines gewichteten Durchschnittswerts (OLD_PEAK) des in Schritt (a) berechneten Mindestwerts (NEW PEAK) und des unmittelbar nach der vorhergehenden EKG-Herzschlagdetektion berechneten gewichteten Durchschnittswerts (OLD_PEAK), wobei der Mindestwert (NEW_PEAK) und der gewichtete Durchschnittswert (OLD_PEAK) jeweils mit jeweiligen vordefinierten Gewichtungsfaktoren (w1, w2) gewichtet werden, wobei die vordefinierten Gewichtungsfaktoren (w1, w2) sich auf eins summieren, und
(c) Neuberechnen der oberen Rauschschwelle (NZTHRESH) durch Nehmen des Maximums von entweder einem vordefinierten Mindestschwellenwert (MIN_NZTHRESH) oder dem in Schritt (b) berechneten und um einen zweiten vordefinierten Skalierungsfaktor (f) skalierten gewichteten Durchschnittswert (OLD_PEAK), wobei der zweite vordefinierte Skalierungsfaktor (f) größer als null und kleiner als eins ist.

9. Verfahren nach Anspruch 1, wobei EKG-Spitzendetektionen unmittelbar vor und unmittelbar nach einer Abtastung, für die eine niedrige Signalqualität angezeigt wurde, als unzuverlässig bezeichnet werden.

**10.** Verfahren nach Anspruch 1, wobei die Rauschbedingungen von Abtastungen auf Basis eines bewegten Fensters bewertet werden, so dass, falls die Anzahl von Abtastungen in irgendeinem Fenster, das eine vordefinierte Anzahl (Y) von Abtastungen enthält, für die eine niedrige Signalqualität bestimmt wird, eine weitere vordefinierte Anzahl (X) übersteigt, EKG-Herzschlagdetektionen unmittelbar vor und nach dem Fenster als unzuverlässig bezeichnet werden.

**11.** Verfahren nach Anspruch 2, wobei das genannte Verfahren die folgenden Schritte umfasst

- Bereitstellen einer Abtastungszahl (NZDW), einer oberen Rauschschwelle (NZTHRESH) als obere Schwelle und einer unteren Rauschschwelle (-NZTHRESH) als untere Schwelle,
- Definieren eines Qualitätskontrollfensters, das eine Anzahl (NZDW) von Abtastungen mit wenigstens einer Abtastung (k) aufweist, wobei die genannte Anzahl der genannten Abtastungszahl entspricht,
- Bestimmen einer Summe überschwelliger Abtastungen (STS_SUM), die der Anzahl von Abtastungen in dem genannten Qualitätskontrollfenster entspricht, die eine Amplitude haben, die die genannte obere Rauschschwelle (NZTHRESH) übersteigt bzw. unter die genannte untere Rauschschwelle (-NZTHRESH) fällt,
- Bestimmen einer Rauschschwellenübergangssumme (NZX_SUM), die der Summe der Schwellenübergangszahl (NZX_CNT) von Rauschschwellenübergängen zwischen jeweils zwei aufeinanderfolgenden Abtastungen im Qualitätskontrollfenster entsprechen,
- Anzeigen einer niedrigen Signalqualität für jede letzte Abtastung (k) jedes Rauschdetektionsfensters, falls

- die genannte Rauschschwellenübergangssumme (NZX_SUM) einen ersten vordefinierten Wert (TX1) übersteigt oder
- die genannte Summe überschwelliger Abtastungen (STS_SUM) einen zweiten vordefinierten Wert (ST1) übersteigt oder
- die genannte Rauschschwellenübergangssumme (NZX SUM) einen dritten vordefinierten Wert (TX2) übersteigt und die genannte Summe überschwelliger Abtastungen (STS_SUM) einen vierten vordefinierten Wert (ST2) übersteigt.

**12.** Verfahren nach Anspruch 3, wobei die Rauschschwellenübergangssumme ein Komplexitätsindex ist, wobei das genannte Verfahren ferner die folgenden Schritte aufweist
Vergleichen des genannten Komplexitätsindexes mit einer Komplexitätsindexschwelle und
Erzeugen eines eine niedrige Signalqualität anzeigenden Signals, falls der genannte Komplexitätsindex die genannte Komplexitätsindexschwelle übersteigt.

**13.** Medizinische Vorrichtung mit einem Mittel zum Verarbeiten eines abgetasteten kardialen elektrischen Signals und einer Verarbeitungseinheit, wobei das genannte kardiale elektrische Signal eine aufeinanderfolgende Folge von Abtastungen umfasst, wobei jede Abtastung eine Amplitude hat, wobei die Amplituden allgemein positiv oder negativ sind, wobei die medizinische Vorrichtung einen Speicher aufweist, wobei der genannte Speicher Daten aufweist, die ein Signalqualitätskontrollfenster definieren, das eine Anzahl aufeinanderfolgender Abtastungen, wenigstens eine obere Schwelle für eine Signalamplitude und wenigstens eine untere Schwelle für eine Signalamplitude, wobei die genannte wenigstens eine obere Schwelle und die genannte wenigstens eine untere Schwelle wenigstens drei Amplitudenbereiche definieren, einen hohen Bereich für Signalamplituden, die die genannte obere Schwelle übersteigen, einen mittleren Bereich für Amplituden zwischen der genannten oberen Schwelle und der genannten unteren Schwelle und einen unteren Bereich für Amplituden unter der genannten unteren Schwelle, wobei die genannte Verarbeitungseinheit konfiguriert ist

- zum Vergleichen jeder Abtastung innerhalb des genannten Qualitätskontrollfensters mit der genannten oberen Schwelle und mit der genannten unteren Schwelle; und
- zum Durchführen einer Signalqualitätsanalyse für die genannte Signalabtastung durch: a) Analysieren der Amplitudenänderungen aufeinanderfolgender Abtastungen innerhalb des genannten Qualitätskontrollfensters, wobei die genannte Amplitudenänderung für beliebige zwei benachbarte Abtastungspaare innerhalb des genannten Qualitätskontrollfensters bestimmt wird durch Vergleichen, zu welchen Amplitudenbereichen die Amplituden der Signalabtastungen des jeweiligen Abtastungspaares gehören, und durch b) Bestimmen einer Schwellenübergangszahl, die die Anzahl von Schwellen zwischen beliebigen zwei benachbarten Signalabtastungen jedes Abtastungspaares reflektieren.

**14.** Medizinische Vorrichtung nach Anspruch 13, wobei die Verarbeitungseinheit ferner konfiguriert ist zum Durchführen einer EKG-Herzschlagdetektion durch Bestimmen, ob eine Amplitude einer beliebigen Abtastung eine Herzschlagdetektionsschwelle übersteigt, und Erzeugen eines Sense-Markers an dieser Abtastung, und zum Bestimmen eines mit jeder EKG-Herzschlagerkennung assoziierten Spitzenwerts (PEAK) durch Definieren eines Spitzendetektionsfensters (PKDW) um den Sense-Marker, das eine vorbestimmte Länge und Position in Bezug auf den Sense-Marker hat, und Bestimmen einer maximalen absoluten Amplitude aller Abtastungen im Spitzendetektionsfenster (PKDW) als einen Spitzenwert (PEAK).

15. Medizinische Vorrichtung nach Anspruch 13, wobei die Verarbeitungseinheit ferner konfiguriert ist zum Neuberechnen der wenigstens einen oberen Schwelle (NZTHRESH; HITHRESH, LOTHRESH) nach einer EKG-Herzschlagdetektion, wobei jede EKG-Herzschlagdetektion einen Spitzenwert (PEAK) ergibt, der einer während eines Spitzendetektionsfensters (PKDW) gemessenen maximalen absoluten Amplitude entspricht, durch Durchführen der folgenden Schritte:

(a) Berechnen eines Mindestwerts (NEW _PEAK) durch Nehmen des Minimums eines Spitzenwerts (PEAK) einer kürzlich durchgeführten EKG-Herzschlagdetektion und eines gewichteten Durchschnittswerts (OLD _PEAK), der unmittelbar nach der vorhergehenden EKG-Herzschlagdetektion berechnet und um einen ersten vordefinierten Skalierungsfaktor (p), der größer als eins ist, skaliert wird,
(b) Berechnen eines gewichteten Durchschnittswerts (OLD _PEAK) des in Schritt (a) berechneten Mindestwerts (NEW PEAK) und des unmittelbar nach der vorhergehenden EKG-Herz schlagdetektion berechneten gewichteten Durchschnittswerts (OLD_PEAK), wobei der Mindestwert (NEW _PEAK) und der gewichtete Durchschnittswert (OLD _PEAK) jeweils mit jeweiligen vordefinierten Gewichtungsfaktoren (w1, w2) gewichtet werden, wobei die vordefinierten Gewichtungsfaktoren (w1, w2) sich auf eins summieren, und
(c) Neuberechnen der wenigstens einen oberen Rauschschwelle (NZTHRESH; HITHRESH, LOTHRESH) durch Nehmen des Maximums von entweder einem vordefinierten Mindestschwellenwert (MIN_NZTHRESH; MIN HITHRESH, MIN LOTHRESH) oder dem in Schritt (b) berechneten und um einen zweiten vordefinierten Skalierungsfaktor (f; fh, fl) skalierten gewichteten Durchschnittswert (OLD_PEAK), wobei der zweite vordefinierte Skalierungsfaktor (f; fh, fl) größer als null und kleiner als eins ist.

16. Medizinische Vorrichtung nach Anspruch 13, wobei die Verarbeitungseinheit ferner konfiguriert ist zum Bewerten der Signalqualität von Signalabtastungen auf Basis eines bewegten Fensters, so dass, falls die Anzahl von Abtastungen in irgendeinem Qualitätskontrollfenster, das eine vordefinierte Anzahl (Y) von Abtastungen enthält, für die eine niedrige Signalqualität bestimmt wird, eine weitere vordefinierte Anzahl (X) übersteigt, EKG-Herzschlagdetektionen unmittelbar vor und nach dem Fenster als unzuverlässig bezeichnet werden.

## Revendications

1. Procédé mis en œuvre par ordinateur permettant la détermination d'un bruit dans un signal électrique cardiaque d'échantillon, ledit signal électrique cardiaque d'échantillon comprenant une séquence consécutive d'échantillons, chaque échantillon ayant une amplitude, les amplitudes étant en général positives ou négatives, le procédé comprenant les étapes

- de mise en place d'une fenêtre de vérification de la qualité du signal qui comprend un nombre d'échantillons consécutifs
- de mise en place d'au moins un seuil supérieur pour une amplitude de signal
- de mise en place d'au moins un seuil inférieur pour une amplitude de signal
- ledit au moins un seuil supérieur et ledit au moins un seuil inférieur définissant au moins trois plages d'amplitudes, une plage haute pour une amplitude de signal excédant ledit seuil supérieur, une plage moyenne pour des amplitudes entre ledit seuil supérieur et ledit seuil inférieur et une plage basse pour une amplitude en-dessous dudit seuil inférieur
- de comparaison de chaque échantillon dans ladite fenêtre de vérification de la qualité avec ledit seuil supérieur et avec ledit seuil inférieur ; et
- de réalisation d'une analyse de qualité du signal pour ledit échantillon de signal en :

a) analysant les variations en amplitude d'échantillons successifs dans ladite fenêtre de vérification de la qualité, ladite variation en amplitude étant déterminée pour toute paire de deux échantillons adjacents dans la fenêtre de vérification de la qualité en comparant à quelles plages d'amplitudes les amplitudes des échantillons de signal de la paire d'échantillons respective correspond, et en
b) déterminant un nombre de franchissements de seuil reflétant le nombre de seuils entre deux échantillons de signal adjacents de chaque paire d'échantillons.

2. Procédé selon la revendication 1, comprenant la détermination d'une somme de franchissements du seuil de bruit desdits nombres de franchissements de seuil pour une fenêtre de vérification de la qualité respective.

3. Procédé selon la revendication 2, dans lequel la somme de franchissements de seuil de bruit est une somme pondérée avec des pondérations prédéterminées pour chaque taille du nombre de franchissements de seuil.

4. Procédé selon la revendication 1, dans lequel le signal cardiaque électrique est un signal d'électrocardiogramme (ECG), un signal d'électrocardiogramme de surface, un signal d'électrocardiogramme sous-cutané ou un signal d'électro-gramme intracardiaque.

5. Procédé selon la revendication 1, dans lequel les grandeurs de l'au moins un premier seuil (NZTHRESH ; HITHRESH ; LOTHRESH) et de l'au moins un seuil inférieur (-NZTHRESH ; -HITHRESH ; -LOTHRESH) sont identiques ; et/ou le seuil de bruit supérieur (NZTHRESH ; HITHRESH ; LOTHRESH) est positif et le seuil de bruit inférieur (-NZTHRESH ; -HITHRESH ; -LOTHRESH) est négatif.

6. Procédé selon la revendication 1, dans lequel une détection de battement d'ECG est effectuée en déterminant si une amplitude d'un échantillon quelconque dépasse un seuil de détection de battement, et en générant un marqueur de sens au niveau de cet échantillon, et dans lequel une fenêtre de détection de pic (PKDW), autour du marqueur de sens, ayant une longueur et une position prédéfinies par rapport au marqueur de sens, est définie, et dans lequel une amplitude absolue maximale de tous les échantillons dans la fenêtre de détection de pic (PKDW) est déterminée sous forme d'une valeur de pic (PEAK).

7. Procédé selon la revendication 1, dans lequel l'au moins un seuil supérieur (NZTHRESH; HITHRESH; LOTHRESH) et/ou le seuil de bruit inférieur (-NZTHRESH; -HITHRESH; -LOTHRESH) sont recalculés après chaque détection de battement d'ECG.

8. Procédé selon la revendication 7, dans lequel l'au moins un seuil de bruit supérieur (NZTHRESH ; HITHRESH) est recalculé après une détection de battement d'ECG, chaque détection de battement d'ECG donnant lieu à une valeur de pic (PEAK) correspondant à une amplitude absolue maximale mesurée durant une fenêtre de détection de pic (PKDW) en effectuant les étapes suivantes :

a) de calcul d'une valeur minimale (NEW _PEAK) en prenant le minimum d'une valeur de pic (PEAK) de la détection de battement d'ECG effectuée récemment, et une valeur moyenne pondérée (OLD _PEAK) calculée directement après la détection de battement d'ECG précédente et étant mise à l'échelle par un premier facteur d'échelon (p) prédéfini supérieur à 1,
b) de calcul d'une valeur moyenne pondérée (OLD _PEAK) de la valeur minimale (NEW PEAK) calculée dans l'étape a), et de la valeur moyenne pondérée (OLD _PEAK) calculée directement après la détection de battement d'ECG précédente, la valeur minimale (NEW PEAK) et la valeur moyenne pondérée (OLD _PEAK) étant chacune pondérée par des facteurs de pondération (w1, w2) prédéfinis respectifs, les facteurs de pondération (w1, w2) prédéfinis ayant pour somme un, et
c) de recalcul du seuil de bruit supérieur (NZTHRESH) en prenant le maximum soit d'une valeur de seuil minimale prédéfinie (MIN_NZTHRESH), soit la valeur moyenne pondérée (OLD _PEAK) calculée dans l'étape (b) et étant mise à l'échelle par un deuxième facteur d'échelon (f) prédéfini, le deuxième facteur d'échelon (f) prédéfini étant supérieur à zéro et inférieur à un.

9. Procédé selon la revendication 1, dans lequel les détections de pic d'ECG immédiatement avant et immédiatement après un échantillon pour lequel une qualité de signal faible a été indiquée, sont étiquetées comme non fiables.

10. Procédé selon la revendication 1, dans lequel les conditions de bruit des échantillons sont évaluées sur une base de fenêtre mobile de sorte que si le nombre d'échantillons dans une fenêtre quelconque contenant un nombre (Y) prédéfini d'échantillons pour lesquels une qualité de signal faible est déterminée, dépasse un autre nombre (X) prédéfini, les détections de battement d'ECG immédiatement avant et après la fenêtre sont étiquetées comme non fiables.

11. Procédé selon la revendication 2, ledit procédé comprenant les étapes :

- de mise en place d'un numéro d'échantillon (NZDW), d'un seuil de bruit supérieur (NZTHRESH) en tant que seuil supérieur, et d'un seuil de bruit inférieur (-NZTHRESH) en tant que seuil inférieur,
- de définition d'une fenêtre de vérification de la qualité comprenant un nombre (NZDW) d'échantillons comportant un dernier échantillon (k), ledit nombre correspondant audit nombre d'échantillon,
- de détermination d'une somme d'échantillons supraliminaire (STS_SUM) correspondant au nombre d'échantillons dans ladite fenêtre de vérification de la qualité qui ont une amplitude qui, soit dépasse ledit seuil de bruit supérieur (NZTHRESH), soit tombe en-dessous dudit seuil de bruit inférieur (-NZTHRESH), respectivement,
- de détermination d'une somme de franchissements de seuil (NZX SUM) correspondant à la somme du nombre de franchissements de seuil (NZX CNT) de franchissements de seuil de bruit entre chacun de deux échantillons consécutifs dans la fenêtre de vérification de la qualité,
- d'indication d'une qualité de signal faible pour chaque dernier échantillon (k) de chaque fenêtre de détection de bruit si

- ladite somme de franchissements de seuil de bruit (NZX SUM) dépasse une première valeur prédéfinie (TX1), ou
- ladite somme d'échantillons supraliminaires (STS_SUM) dépasse une deuxième valeur prédéfinie (ST1), ou
- ladite somme de franchissements de seuil de bruit (NZX SUM) dépasse une troisième valeur prédéfinie (TX2) et ladite somme d'échantillons supraliminaires (STS_SUM) dépasse une quatrième valeur prédéfinie (ST2).

**12.** Procédé selon la revendication 3, dans lequel la somme de franchissements de seuil de bruit est un indice de complexité, ledit procédé comprenant en outre les étapes de comparaison dudit indice de complexité avec un seuil d'indice de complexité et de génération d'un signal indiquant une qualité de signal faible si ledit indice de complexité dépasse ledit seuil d'indice de complexité.

**13.** Dispositif médical ayant des moyens de traitement d'un signal électrique cardiaque d'échantillons et une unité de traitement, ledit signal électrique cardiaque comprenant une séquence consécutive d'échantillons, chaque échantillon ayant une amplitude, les amplitudes étant en général positives ou négatives, où le dispositif médical comprend une mémoire, ladite mémoire comprenant des données définissant une fenêtre de vérification de la qualité du signal qui comprend un nombre d'échantillons consécutifs, au moins un seuil supérieur pour une amplitude de signal et au moins un seuil inférieur pour une amplitude de signal, ledit au moins un seuil supérieur et ledit au moins un seuil inférieur définissant au moins trois plages d'amplitudes, une plage haute pour une amplitude de signal excédant ledit seuil supérieur, une plage moyenne pour des amplitudes entre ledit seuil supérieur et ledit seuil inférieur et une plage basse pour une amplitude en-dessous dudit seuil inférieur, où ladite unité de traitement est conçue

- pour comparer chaque échantillon dans ladite fenêtre de vérification de la qualité avec ledit seuil supérieur et avec ledit seuil inférieur ; et
- pour effectuer une analyse de la qualité du signal pour ledit échantillon de signal en :

a) analysant les variations en amplitude d'échantillons successifs dans ladite fenêtre de vérification de la qualité, ladite variation en amplitude étant déterminée pour toute paire de deux échantillons adjacents dans la fenêtre de vérification de la qualité en comparant à quelles plages d'amplitudes les amplitudes des échantillons de signal de la paire d'échantillons respective correspond, et en
b) déterminant un nombre de franchissements de seuil reflétant le nombre de seuils entre deux échantillons de signal adjacents de chaque paire d'échantillons.

**14.** Dispositif médical selon la revendication 13, dans lequel l'unité de traitement est en outre conçue pour effectuer une détection de battement d'ECG en déterminant si une amplitude d'un échantillon quelconque dépasse un seuil de détection de battement, et en générant un marqueur de sens au niveau de cet échantillon, et pour déterminer une valeur de pic (PEAK) associée avec chaque détection de pic d'ECG, en définissant une fenêtre de détection de pic (PKDW), autour du marqueur de sens, ayant une longueur et une position prédéfinies par rapport au marqueur de sens, et en déterminant une amplitude absolue maximale de tous les échantillons dans la fenêtre de détection de pic (PKDW) sous forme d'une valeur de pic (PEAK).

**15.** Dispositif médical selon la revendication 13, dans lequel l'unité de traitement est en outre conçue pour recalculer l'au moins un premier seuil (NZTHRESH ; HITHRESH ; LOTHRESH) après une détection de battement d'ECG, chaque détection de battement d'ECG donnant lieu à une valeur de pic (PEAK) correspondant à une amplitude absolue maximale mesurée durant une fenêtre de détection de pic (PKDW) en effectuant les étapes suivantes :

a) de calcul d'une valeur minimale (NEW_PEAK) en prenant le minimum d'une valeur de pic (PEAK) de la détection de battement d'ECG effectuée récemment, et une valeur moyenne pondérée (OLD_PEAK) calculée directement après la détection de battement d'ECG précédente et étant mise à l'échelle par un premier facteur d'échelon (p) prédéfini supérieur à 1,

b) de calcul d'une valeur moyenne pondérée (OLD_PEAK) de la valeur minimale (NEW_PEAK) calculée dans l'étape a), et la valeur moyenne pondérée (OLD_PEAK) calculée directement après la détection de battement d'ECG précédente, la valeur minimale (NEW_PEAK) et la valeur moyenne pondérée (OLD_PEAK) étant chacune pondérée par des facteurs de pondération (w1, w2) prédéfinis respectifs, les facteurs de pondération (w1, w2) prédéfinis ayant pour somme un, et

c) de recalcul de l'au moins un seuil de bruit supérieur (NZTHRESH ; HITHRESH ; LOTHRESH) en prenant le maximum soit d'une valeur de seuil minimale prédéfinie (MIN_NZTHRESH ; MIN_HITHRESH ; MIN_LOTHRESH), soit la valeur moyenne pondérée (OLD_PEAK) calculée dans l'étape (b) et étant mise à l'échelle par un deuxième facteur d'échelonnement (f ; fh ; fi) prédéfini, le deuxième facteur d'échelonnement (f ; fh ; fi) prédéfini étant supérieur à zéro et inférieur à un.

16. Dispositif médical selon la revendication 13, dans lequel l'unité de traitement est en outre conçue pour évaluer la qualité du signal sur une base de fenêtre mobile de sorte que si le nombre d'échantillons dans une quelconque fenêtre de vérification de la qualité contenant un nombre (Y) prédéfini d'échantillons, pour lesquels une faible qualité de signal est déterminée, dépasse un autre nombre (X) prédéfini, les détections de battement d'ECG immédiatement avant et après la fenêtre sont étiquetées comme non fiables.

FIG. 1

FIG. 2

**FIG. 3**

EP 2 407 097 B1

**FIG. 4**

NZX_CNT    0    0    0    1    1    1    2    0    0    2    1    1

NTZ

NZTHRESH

-NZTHRESH

**FIG. 5A**

STS_CNT    0    0    0    1    0    1    1    1    1    1    0    1

NTZ

NZTHRESH

-NZTHRESH

**FIG. 5B**

EP 2 407 097 B1

**FIG. 6**

FIG. 7

EP 2 407 097 B1

FIG. 8

**FIG. 9**

PKDW

sense

PKDW

sense

PKDW

sense

HITHRESH

LOTHRESH

-LOTHRESH

-HITHRESH

A  B  C  D  E

**FIG. 10**

FIG. 11

EP 2 407 097 B1

Symbol   c   c   b   b   d   a   b   e   c   a   a   e   e   a   c

**FIG. 12**

EP 2 407 097 B1

HITHRESH
LOTHRESH
-LOTHRESH
-HITHRESH

15

10

5

0

0    1    2    3    4

Absolute symbol change step

FIG. 13

HITHRESH
LOTHRESH
-LOTHRESH
-HITHRESH

CI

**FIG. 14**

EP 2 407 097 B1

**EP 2 407 097 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5891048 A
- US 7496409 B, Greenhut
- US 6230059 B, Duffin
- US 7467009 B
- US 6917830 B, Palreddy
- US 7474247 B, Hinks
- US 7027858 B, Cao
- US 20080161873 A, Gunderson
- US 20080300497 A, Krause
- US 2008269813 A, Greenhut
- US 2007255150 A, Brodnick
- US 6345199 B, Thong
- US 7570989 B, Baura
- US 5999845 A, dePinto
- US 5647379 A, Meltzer
- US 20100312131 A1, Naware
- US 6321115 B, Mouchawar
- US 20080281216 A1
- US 6470215 B
- US 6574509 B
- US 6622043 B